# EUROPEAN PATENT SPECIFICATION

(11) **EP 2 586 861 B1**
(45) Date of publication and mention of the grant of the patent: **25.10.2017**
(21) Application number: 11798167.0
(22) Date of filing: 22.06.2011
(51) Int. Cl.: C12N 15/09, C12M 1/00, C12Q 1/68, B01L 3/02, B01L 3/00, B01L 7/00, C12P 19/34, C08L 91/00, C08L 91/06

(54) **COMPOSITION FOR PREVENTING EVAPORATION OF REACTION SOLUTION DURING NUCLEIC ACID AMPLIFICATION REACTION**
ZUSAMMENSETZUNG ZUR VERHINDERUNG DER VERDAMPFUNG EINER REAKTIONSLÖSUNG WÄHREND EINER NUKLEINSÄURE-AMPLIFIKATIONSREAKTION
COMPOSITION POUR EMPÊCHER L'ÉVAPORATION D'UNE SOLUTION RÉACTIONNELLE AU COURS D'UNE RÉACTION D'AMPLIFICATION D'ACIDES NUCLÉIQUES

(30) Priority: 22.06.2010 JP 2010141510
(43) Date of publication of application: 01.05.2013
(73) Proprietor: Universal Bio Research Co., Ltd., Matsudo-shi Chiba 271-0064 (JP)
(72) Inventor: SUGIMOTO, Takaki, Matsudo-shi Chiba 271-0064 (JP); UEDA, Tetsuya, Matsudo-shi Chiba 271-0064 (JP); SEGAWA, Osamu, Matsudo-shi Chiba 271-0064 (JP); KOIZUKA, Michinori, Matsudo-shi Chiba 271-0064 (JP); TAJIMA, Hideji, Matsudo-shi Chiba 271-0064 (JP)
(74) Representative: J A Kemp
(86) International application number: PCT/JP2011/064232
(87) International publication number: WO 2011/162285

(56) References cited:
- JP-A- 2001 136 964
- JP-A- 2006 349 557
- JP-A- 2006 349 558
- JP-A- 2007 175 006
- US-A- 5 411 876
- US-A- 5 576 197
- US-A1- 2006 204 997
- US-A1- 2009 246 782
- MICROMACHINE GIJUTSU SORAN HENSHU IINKAI MICROMACHINE GIJUTSU SORAN 22 January 2003, pages 729 - 731
- JAPAN OIL CHEMISTS' SOCIETY YUSHI KAGAKU BENRAN 28 February 1990, pages 99 - 101

## Description

### TECHNICAL FIELD

The present invention relates to a method of nucleic acid extraction, amplification and detection in which evaporation of the nucleic acid amplification reaction solution is prevented during the reaction; and an apparatus which performs extraction of nucleic acid from a sample, amplification of the nucleic acid and detection of the nucleic acid in a continuous manner.

### BACKGROUND ART

Recently, genetic analyses have been performed in wide variety of fields such as medicine, agriculture, physics and pharmacology for a diversity of purposes, e.g., genome sequencing, clinical diagnosis, cultivar improvement in agricultural products, bacteria test in foods, drug development and so forth. In genetic analysis which is applicable to such a wide variety of fields with great potential, nucleic acid amplification reactions are frequently used.

Among all, polymerase chain reaction (hereinafter, referred to as "PCR") is a technique for amplifying a target nucleic acid by means of temperature swing, using a thermostable polymerase and primers. The principle of PCR is to amplify a target DNA geometrically by repeating a large number of cycles following thermal profiles (temperature swing) set in three stages: 1^{st} stage where the temperature is maintained at about 94°C at which a double-stranded DNA comprising a target DNA sequence is dissociated to single strands, 2^{nd} stage where the temperature is maintained at about 50°C to about 60°C at which forward and reverse primers anneal to the dissociated single-stranded DNA, and 3^{rd} stage where the temperature is maintained at about 74°C at which a DNA strand complementary to the single-stranded DNA is synthesized by a DNA polymerase.

When PCR is used, it is required to prepare a PCR reaction solution containing a target DNA by performing such operations as isolation/purification of cells containing the target DNA and extraction of the target DNA from the cells. Recently, in particular, for efficient handling of a large number of samples in genetic diagnoses and genome projects, a necessity to automate a series of operations (such as isolation/purification of cells containing a target DNA, extraction of the target DNA from the cells, and amplification of the target DNA by PCR) and to thereby handle a large number of samples in parallel and efficiently has been increased.

Automated apparatuses for simultaneous handling of a large number of samples at a time have been developed and used (Patent Documents Nos. 1 and 2). Patent Document No. 3 describes a method of nucleic acid amplification by PCR wherein two aqueous solutions are separated by a solid wax barrier that melts during the PCR reaction, allowing the solutions to mix. Patent Document No. 4 describes a method of nucleic acid amplification by PCR comprising the use of PCR tubes and wax to seal the PCR mixture in the tubes.
[Patent Document No. 1]
   Japanese Patent No. 3115501
[Patent Document No. 2]
   Japanese Patent No. 3630499
[Patent Document No. 3]
   US Patent No. 5411876
[Patent Document No. 4]
   US Patent No. 5576197

### DISCLOSURE OF THE INVENTION

### PROBLEM FOR SOLUTION BY THE INVENTION

Usually, a closure member or adhesive seal is used to hermetically seal a reaction vessel (tube) in an automated apparatus. Installation of such a closure member or adhesive seal in the apparatus makes the apparatus complicated and expensive.

Further, evaporated liquid generated by heating the reaction vessel (tube) adheres to the closure member or adhesive seal and makes them cloudy. As a result, optical measurement from above the reaction vessel (tube) becomes difficult. To avoid this problem, a hot plate is necessary but then the apparatus becomes more complicated.

It is an object of the present disclosure to provide a composition which is capable of hermetically seal a reaction vessel without using a closure member or adhesive seal.

It is an object of the present invention to provide a method of nucleic acid extraction, amplification and detection, wherein evaporation of the nucleic acid amplification reaction solution is prevented during the amplification reaction.

It is still another object of the present disclosure to provide a prepackaged reagent containing a composition for preventing evaporation of a nucleic acid amplification reaction solution during the amplification reaction.

It is an object of the present invention to provide an apparatus which performs extraction of nucleic acid from a sample, amplification of the nucleic acid and detection of the nucleic acid in a continuous manner.

### MEANS TO SOLVE THE PROBLEM

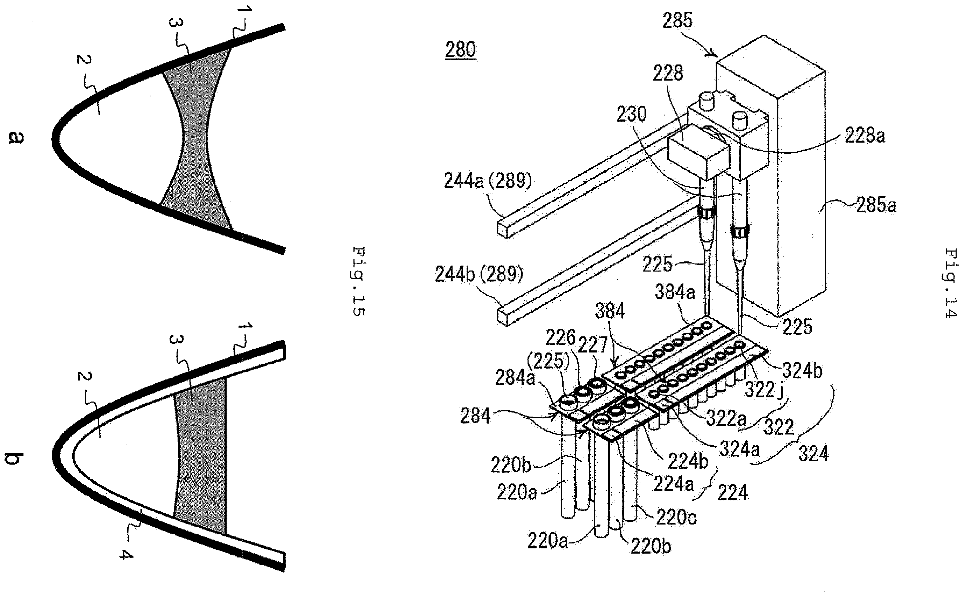

The present inventors have succeeded in assuring hermetical sealing at least comparable to the hermetical sealing of a closure member or a seal by dispensing an oily component (such as mineral oil) into a reaction vessel with a dispenser or the like to thereby confine the reaction solution therein and, at the same time, succeeded in preventing the occurrence cloudiness on heating by allowing no gas to be present between the reaction solution and the oily component. Further, by employing as the oily component a composition which is a liquid during nucleic acid amplification reaction and becomes a solid through chemical changes or thermal changes after completion of the reaction, it has become possible to solidify the oily component after completion of the reaction to thereby (a) facilitate disposal of the reaction solution containing a sample and (b) prevent pollution or contamination resulting from scattering of the reaction solution, operational errors, etc. Further, by coating the inner surface of a reaction vessel with a material having a small surface tension, it has become possible to flatten the surface of the oily component (Fig. 15b). As a result, it has become possible to increase the efficiency of irradiation and/or light reception from above the reaction vessel by preventing light scattering and increasing the area of uniform light reception. The present invention has been achieved based on these findings.

The present disclosure may be summarized as follows.
(1) A composition for preventing evaporation of a nucleic acid amplification reaction solution during nucleic acid amplification reaction, which is a liquid during the reaction and becomes a solid through chemical or thermal changes after completion of the reaction.
(2) A composition for preventing evaporation of a nucleic acid amplification reaction solution during nucleic acid amplification reaction, wherein the melting point of the composition is 0-15°C.
(3) A composition for preventing evaporation of a nucleic acid amplification reaction solution during nucleic acid amplification reaction, wherein the melting point of the composition is 5-10°C.
(4) A method of nucleic acid amplification, comprising a step of layering the composition according to any one of (1) to (3) above on top of the nucleic acid amplification reaction solution.
(5) A method of nucleic acid amplification, comprising a step of solidifying the composition according to any one of (1) to (3) above after completion of the nucleic acid amplification reaction.
(6) A method of nucleic acid amplification, which uses a combination of a composition for preventing evaporation of a nucleic acid amplification reaction solution and a nucleic acid amplification reaction vessel where the shape of the interface to be formed between the composition and air is level or upwardly convex.
(7) A method of nucleic acid amplification, which uses a combination of a composition for preventing evaporation of a nucleic acid amplification reaction solution and a nucleic acid amplification reaction vessel where the wetting tension of its inner surface is smaller than the surface tension of the composition.
(8) A method of nucleic acid amplification, which uses a combination of a composition for preventing evaporation of a nucleic acid amplification reaction solution and a nucleic acid amplification reaction vessel where the wetting tension of its inner surface is smaller than 80% of the surface tension of the composition.
(9) A prepackaged reagent for nucleic acid amplification, containing a composition for preventing evaporation of a nucleic acid amplification reaction solution, wherein the reagent comprises the composition according to any one of (1) to (3) above.
(10) A prepackaged reagent for nucleic acid amplification, containing a composition for preventing evaporation of a nucleic acid amplification reaction solution and a reaction vessel, wherein the combination of the reaction vessel and the composition for preventing evaporation of the reaction solution is the combination according to any one of (6) to (8) above.
(11) An apparatus which performs extraction of nucleic acid from a sample, amplification of the nucleic acid and detection of the nucleic acid in a continuous manner, wherein the nucleic acid reaction solution is hermetically sealed with a composition for preventing evaporation of the reaction solution at the steps of amplification and detection and wherein it possible to optically detect amplification of the nucleic acid through the composition.
(12) An apparatus which performs extraction of nucleic acid from a sample, amplification of the nucleic acid and detection of the nucleic acid in a continuous manner, wherein the nucleic acid reaction solution is hermetically sealed with a composition for preventing evaporation of the reaction solution at the steps of amplification and detection and wherein it is possible to prevent leakage and scattering of the reaction solution by solidifying the composition after completion of the reaction.
(13) An apparatus which performs extraction of nucleic acid from a sample, amplification of the nucleic acid and detection of the nucleic acid in a continuous manner, wherein it is possible to prevent evaporation of the reaction solution with the composition according to any one of (1) to (3) above.
(14) An apparatus which performs extraction of nucleic acid from a sample, amplification of the nucleic acid and detection of the nucleic acid in a continuous manner, wherein the apparatus uses a combination of a reaction vessel and a composition for preventing evaporation of the reaction solution, the combination being the combination according to any one of (6) to (8) above.
(15) An apparatus which performs extraction of nucleic acid from a sample, amplification of the nucleic acid and detection of the nucleic acid in a continuous manner, wherein the apparatus is capable of containing the prepackaged reagent according to (9) or (10) above.

The present invention can be summarized as follows.
(1) A method of nucleic acid extraction, amplification and detection, comprising the steps of providing a liquid composition which becomes a solid through chemical or thermal changes, overlayering the liquid composition on top of a nucleic acid amplification reaction solution before a nucleic acid amplification reaction, performing the nucleic acid amplification reaction, and solidifying the composition after completion of the nucleic acid amplification reaction,
   which uses a nucleic acid amplification reaction vessel where the wetting tension of the inner surface of the reaction vessel is smaller than the surface tension of said composition, wherein the shape of the interface to be formed between the composition and air is level or upwardly convex, and wherein detection of the amplification of the nucleic acid is carried out through the composition by irradiation and/or light reception from above the reaction vessel.
(2) The method of (1) wherein the wetting tension of the inner surface of the reaction vessel is smaller than 80% of the surface tension of said composition.
(3) The method of (1) wherein the reaction vessels are made of polytetrafluoroethylene, tetrafluoroethylene/perfluoroalkylvinyl ether copolymer, or tetrafluoroethylene/ethylene copolymer.
(4) The method of (1) wherein the inner surface of the reaction vessels is coated with a coating agent selected from fluoroacrylic resin, fluorosilane or another fluorine-loaded synthetic resin.
(5) An apparatus which performs extraction of nucleic acid from a sample, amplification of the nucleic acid and detection of the nucleic acid in a continuous manner, which apparatus comprises:
   (a) means for depositing a liquid composition which becomes solid through chemical or thermal changes;
   (b) a nucleic acid reaction vessel where the wetting tension of the inner surface of the reaction vessel is smaller than the surface tension of said composition and where the shape of the interface to be formed between said composition and air is level or upwardly convex; and
   (c) an optical measurement unit;
      wherein the apparatus is capable of overlayering a liquid composition on top of a nucleic acid amplification reaction solution before a nucleic acid amplification reaction, performing the nucleic acid amplification reaction and solidifying the composition after completion of the nucleic acid amplification reaction, the composition becoming a solid through chemical or thermal changes, and wherein the amplification of the nucleic acid can be optically detected through the composition by irradiation and/or light reception from above the reaction vessel, wherein the reaction vessel contains the nucleic acid amplification reaction solution and the liquid composition overlayered on top of the nucleic acid amplification reaction solution, and wherein the shape of the interface formed between the liquid composition and air is level or upwardly convex.
(6) The apparatus of (5) wherein the reaction vessels are made of polytetrafluoroethylene, tetrafluoroethylene/perfluoroalkylvinyl ether copolymer, or tetrafluoroethylene/ethylene copolymer.
(7) The apparatus of (5) wherein the inner surface of the reaction vessels is coated with a coating agent selected from fluoroacrylic resin, fluorosilane or another fluorine-loaded synthetic resin.

### EFFECT OF THE INVENTION

According to the present disclosure, it is possible to prevent evaporation of a nucleic acid amplification reaction solution during amplification reaction. Further, according to the present disclosure, disposal of the reaction solution containing a sample is facilitated and it is possible to prevent pollution or contamination resulting from scattering of the reaction solution, operational errors, etc. Further, according to the present invention, it is possible to increase the efficiency of irradiation and/or light reception from above the reaction vessel by preventing light scattering in the reaction solution and increasing the area of uniform light reception.

The present specification refers to the contents described in the specification and/or drawings of Japanese Patent Application No. 2010-141510 based on which the present application claims priority.

### BRIEF DESCRIPTION OF THE DRAWINGS

FIG. 1 is a perspective view where a part of a housing of a specimen testing device according to a first embodiment is removed.
FIG. 2 is a perspective view where a part of components of the specimen testing device of FIG. 1 is removed and illustrates a state where a test cartridge container is drawn forth.
FIG. 3 is an enlarged perspective view of a component which is built in an optical measurement unit illustrated in FIGS. 1 and 2.
FIG. 4 is an enlarged perspective view of the test cartridge container illustrated in FIGS. 1 and 2.
FIG. 5 is a perspective view illustrating various tips accommodated in the test cartridge container illustrated in FIG. 4.
FIG. 6 is a processing flow view of the specimen testing device illustrated in FIGS. 1 and 2.
FIG. 7 is a perspective view illustrating that main components of a specimen testing device according to a second embodiment are taken out of a housing.
FIG. 8 is a perspective view illustrating that main components of a specimen testing device according to a third embodiment are taken out of a housing.
FIG. 9 is an enlarged perspective view illustrating an optical measurement unit and a temperature controller illustrated in FIG. 8 partially cut out.
FIG. 10 is a perspective view illustrating that main components of a specimen testing device according to a fourth embodiment are taken out of a housing.
FIG. 11 is an enlarged perspective view illustrating an optical measurement unit and a temperature controller illustrated in FIG. 10 partially cut out.
FIG. 12 is an enlarged perspective view illustrating a cap illustrated in FIG. 11.
FIG. 13 is a perspective view of a cap illustrated in FIG. 12 partially cut out.
FIG. 14 is a pattern diagram illustrating that main components including four test cartridge containers of a specimen testing device according to a fifth embodiment are taken out of a housing.
Fig. 15 is a conceptual diagram showing that the surface of oily component is flattened by coating the inner surface of a reaction vessel with a material having a small surface tension. a: A cross section showing the state of reaction solution 2 and oily component 3 in reaction vessel 1 the inner surface of which has no coating. b: A cross section showing the state of reaction solution 2 and oily component 3 in reaction vessel 1 which has coating 4 on its inner surface.
Fig. 16 shows fluorescence intensity distribution in the aperture plane of a reaction vessel. 16-1: Fluorescence intensity distribution in the aperture plane of an untreated reaction vessel. ◆ Untreated vessel: fluorescent aqueous solution alone. ■ Untreated vessel: fluorescent aqueous solution + mineral oil. 16-2: Fluorescence intensity distribution in the aperture plane of a reaction vessel treated with a water- and oil-repellent agent. ◆ Treated vessel: fluorescent aqueous solution alone. ■ Treated vessel: fluorescent aqueous solution + mineral oil.

### BEST MODE FOR CARRYING OUT THE INVENTION

Hereinbelow, embodiments of the present invention will be described in more detail.

The present disclosure provides a composition for preventing evaporation of a nucleic acid amplification reaction solution during nucleic acid amplification reaction, which is a liquid during the reaction and becomes a solid through chemical or thermal changes after completion of the reaction.

The present disclosure also provides a composition for preventing evaporation of a nucleic acid amplification reaction solution during nucleic acid amplification reaction, wherein the melting point of the composition is 0-15°C.

The present disclosure also provides a composition for preventing evaporation of a nucleic acid amplification reaction solution during nucleic acid amplification reaction, wherein the melting point of the composition is 5-10°C.

The composition of the present disclosure may contain mineral oil, silicone oil, other chemically synthesized oil or a combination thereof.

Mineral oil is petroleum-derived oil, and specific examples thereof include liquid paraffin and solid paraffin.

Silicone oil is an oil containing molecules of a linear structure with 2000 or less siloxane bonds, and is roughly divided into straight silicone oil and modified silicone oil. Either of them may be used in the composition of the present disclosure.

In order for the composition of the present disclosure to be a liquid during reaction and to become a solid through chemical changes after completion of the reaction, the constitution of components of the composition may be adjusted to give a melting point that is either at room temperature or below the reaction temperature (e.g., about 50°C) and, in addition, a solidifying agent may be used.

Specific examples of compositions whose melting point is either at room temperature or below the reaction temperature include liquid paraffin and mineral oil.

Specific examples of the solidifying agent include a high melting point (higher than room temperature) paraffin (such as a solid paraffin with a melting point of 44-46°C).

In order for the composition of the present disclosure to be a liquid during reaction and to become a solid through thermal changes after completion of the reaction, the constitution of components of the composition may be adjusted to give a melting point of 0-15 °C, preferably 5-10 °C. For example, it is possible to give the composition a melting point of -10 to 40°C by adding 5.0-15.0 mass percent of solid paraffin (melting point 44-46 °C) to liquid paraffin taken as unity.

The composition of the present disclosure may be added to a reaction vessel in an amount that is barely sufficient to completely cover that surface of the nucleic acid amplification reaction solution in the vessel which is in contact with air. The amount to be added is preferably 1.1 to 3 times, more preferably 1.5 to 2 times, the minimum amount required to completely cover that surface of the nucleic acid amplification reaction solution in the vessel which is in contact with air.

It is possible to prevent evaporation of a nucleic acid amplification reaction solution by layering the composition of the present disclosure on top of the reaction solution. By thus enclosing the reaction solution with the composition of the present disclosure, hermetical sealing at least comparable to the hermetical sealing provided with a closure member or a seal is assured. At the same time, since no gas is allowed to be present between the reaction solution and the composition, the occurrence of cloudiness may be prevented or reduced.

The present disclosure provides a method of nucleic acid amplification, comprising a step of layering the above-described composition on top of the nucleic acid amplification reaction solution.

Further, the present disclosure provides a method of nucleic acid amplification, comprising a step of solidifying the above-described composition after completion of nucleic acid amplification reaction.

By solidifying the above-described composition after completion of nucleic acid amplification reaction, it is possible to facilitate disposal of the reaction solution and to prevent pollution or contamination resulting from scattering of the reaction solution, operational errors, etc. The method of solidification is as described above.

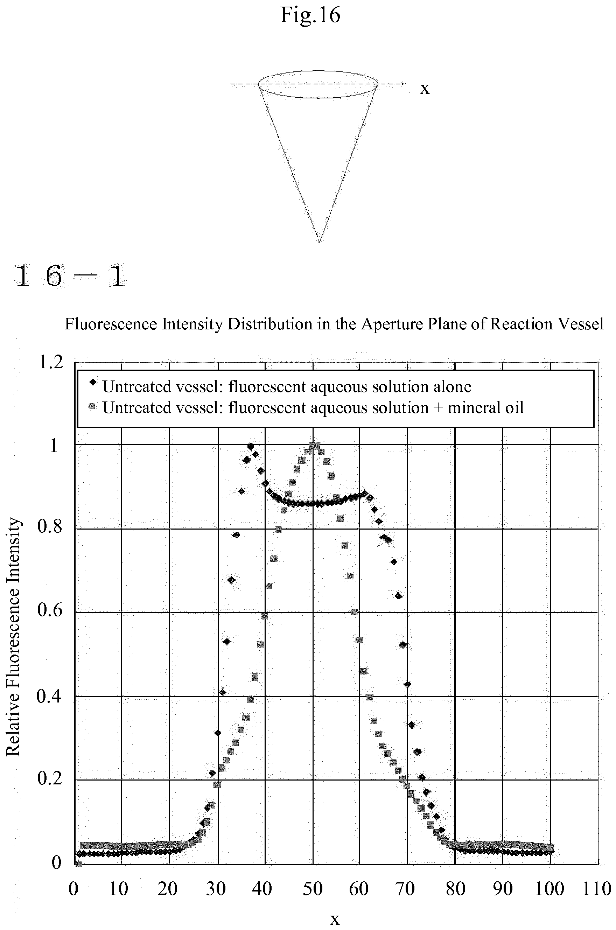

When a composition for preventing evaporation of a nucleic acid amplification reaction solution in a nucleic acid amplification reaction vessel (e.g., oily component such as mineral oil or silicone oil) is layered on the top of reaction solution, the shape of the interface between the composition and air becomes upwardly concave (Fig. 15a). Therefore, light scatters to thereby reduce the area of uniform light reception (Fig. 16-1 ■). As a result, a problem of decrease of accuracy in measurement occurs. In order to solve this problem, the present inventors have used the combinations described below.
- A combination of a composition for preventing evaporation of a nucleic acid amplification reaction solution and a nucleic acid amplification reaction vessel where the shape of the interface to be formed between the composition and air is level or upwardly convex.
- A combination of a composition for preventing evaporation of a nucleic acid amplification reaction solution and a nucleic acid amplification reaction vessel where the wetting tension of its inner surface is smaller than the surface tension of the composition.
- A combination of a composition for preventing evaporation of a nucleic acid amplification reaction solution and a nucleic acid amplification reaction vessel where the wetting tension of its inner surface is smaller than 80% of the surface tension of the composition.

Therefore, the present invention provides a method of nucleic acid extraction, amplification and detection, comprising the steps of providing a liquid composition which becomes a solid through chemical or thermal changes, overlayering the liquid composition on top of a nucleic acid amplification reaction solution before a nucleic acid amplification reaction, performing the nucleic acid amplification reaction, and solidifying the composition after completion of the nucleic acid amplification reaction,
which uses a nucleic acid amplification reaction vessel where the wetting tension of the inner surface of the reaction vessel is smaller than the surface tension of said composition, wherein the shape of the interface to be formed between the composition and air is level or upwardly convex, and wherein detection of the amplification of the nucleic acid is carried out through the composition by irradiation and/or light reception from above the reaction vessel.

Disclosed is a method of nucleic acid amplification, which uses a combination of a composition for preventing evaporation of a nucleic acid amplification reaction solution and a nucleic acid amplification reaction vessel where the shape of the interface to be formed between the composition and air is level or upwardly convex.

Disclosed is a method of nucleic acid amplification, which uses a combination of a composition for preventing evaporation of a nucleic acid amplification reaction solution and a nucleic acid amplification reaction vessel where the wetting tension of its inner surface is smaller than the surface tension of the composition.

A method for testing wetting tension is specified in JIS K6768.

Disclosed is a nucleic acid amplification, which uses a combination of a composition for preventing evaporation of a nucleic acid amplification reaction solution and a nucleic acid amplification reaction vessel where the wetting tension of its inner surface is smaller than 80% of the surface tension of the composition

Reaction vessels where the shape of the interface to be formed between a composition for preventing evaporation of a nucleic acid amplification reaction solution therein and air is level or upwardly convex, reaction vessels where the wetting tension of their inner surface is smaller than the surface tension of a composition for preventing evaporation of a nucleic acid amplification reaction solution, and reaction vessels where the wetting tension of their inner surface is smaller than 80% of the surface tension of a composition for preventing evaporation of a nucleic acid amplification reaction solution include, may be exemplified by those which are made of polytetrafluoroethylene, tetrafluoroethylene/perfluoroalkylvinyl ether copolymer, or tetrafluoroethylene/ethylene copolymer. Other examples are reaction vessels the inner surface of which is coated with a coating agent having a small surface tension (such as fluoroacrylic resin, fluorosilane or other fluorine-loaded synthetic resin).

The reaction vessel may be made of any one of such materials as polypropylene, polycarbonate, polyvinyl chloride, polyester and nylon. The thickness of the coating is not particularly limited; about 1 µm may be appropriate.

When the coating agent is fluorosilane, it is preferable to perform a primer treatment in order to enhance adhesion. As a primer, liquid glass or the like may be given.

The reaction vessel where the shape of the interface to be formed between a composition for preventing evaporation of a nucleic acid amplification reaction solution therein and air is level or upwardly convex may be a reaction vessel where the wetting tension of its inner surface is smaller than the surface tension of the composition for preventing evaporation of the reaction solution, preferably a reaction vessel where the wetting tension of its inner surface is smaller than 80% of the surface tension of the composition for preventing evaporation of the reaction solution.

In the methods of nucleic acid amplification of the present disclosure, a composition for preventing evaporation of the reaction solution may be the composition of the present disclosure as described above. Alternatively, known compositions which have been used for preventing evaporation of reaction solutions (e.g., mineral oil manufactured by Applied Biosystems; oily components disclosed in US Patents Nos. 5411876, 5576197, 5599660, and 5413924 and Japanese Unexamined Patent Publications Nos. 2007-275005 and 2007-175006) may also be used.

Specific examples of the combination of a composition for preventing evaporation of a nucleic acid amplification reaction solution and a nucleic acid amplification reaction vessel where the shape of the interface to be formed between the composition and air is level or upwardly convex include, but are not limited to, a combination of mineral oil manufactured by Applied Biosystems and a Roche PCR tube the inner wall surface of which is coated with a fluorinated coating agent FS-1010 manufactured by Fluoro Technology.

The composition of the present disclosure may be contained in a prepackaged reagent for nucleic acid amplification.

The present disclosure provides a prepackaged reagent for nucleic acid amplification, containing a composition for preventing evaporation of a nucleic acid amplification reaction solution, wherein the reagent comprises any one of the following compositions for preventing evaporation of reaction solution (1) to (3).
(1) A composition for preventing evaporation of a nucleic acid amplification reaction solution during nucleic acid amplification reaction, which is a liquid during the reaction and becomes a solid through chemical or thermal changes after completion of the reaction.
(2) A composition for preventing evaporation of a nucleic acid amplification reaction solution during nucleic acid amplification reaction, wherein the melting point of the composition is 0-15°C.
(3) A composition for preventing evaporation of a nucleic acid amplification reaction solution during nucleic acid amplification reaction, wherein the melting point of the composition is 5-10°C.

The compositions of (1) to (3) are as described above.

The prepackaged reagent of the present disclosure may comprise a reaction vessel. The reaction vessel is as described above.

The present disclosure provides a prepackaged reagent for nucleic acid amplification, containing a composition for preventing evaporation of a nucleic acid amplification reaction solution and a reaction vessel, wherein the combination of the reaction vessel and the composition for preventing evaporation of the reaction solution is any one of the following combinations (1a) to (3a).
(1a) A combination of a composition for preventing evaporation of a nucleic acid amplification reaction solution and a nucleic acid amplification reaction vessel where the shape of the interface to be formed between the composition and air is level or upwardly convex.
(1b) A combination of a composition for preventing evaporation of a nucleic acid amplification reaction solution and a nucleic acid amplification reaction vessel where the wetting tension of its inner surface is smaller than the surface tension of the composition.
(1c) A combination of a composition for preventing evaporation of a nucleic acid amplification reaction solution and a nucleic acid amplification reaction vessel where the wetting tension of its inner surface is smaller than 80% of the surface tension of the composition.

The composition and the reaction vessel of (1a) to (1c) are as described above.

The prepackaged reagent of the present disclosure may further comprise nucleic acid extraction reagents, nucleic acid amplification reaction solutions, and so forth.

The composition, the prepackaged reagent and the method of nucleic acid amplification of the present disclosure may be applicable to both manual operations and automated nucleic acid amplification apparatuses.

The present invention also provides an apparatus which performs extraction of nucleic acid from a sample, amplification of the nucleic acid and detection of the nucleic acid in a continuous manner, which apparatus comprises:
(a) means for depositing a liquid composition which becomes solid through chemical or thermal changes;
(b) a nucleic acid reaction vessel where the wetting tension of the inner surface of the reaction vessel is smaller than the surface tension of said composition and where the shape of the interface to be formed between said composition and air is level or upwardly convex; and
(c) an optical measurement unit;
wherein the apparatus is capable of overlayering a liquid composition on top of a nucleic acid amplification reaction solution before a nucleic acid amplification reaction, performing the nucleic acid amplification reaction and solidifying the composition after completion of the nucleic acid amplification reaction, the composition becoming a solid through chemical or thermal changes, and wherein the amplification of the nucleic acid can be optically detected through the composition by irradiation and/or light reception from above the reaction vessel, wherein the reaction vessel contains the nucleic acid amplification reaction solution and the liquid composition overlayered on top of the nucleic acid amplification reaction solution, and wherein the shape of the interface formed between the liquid composition and air is level or upwardly convex.

Disclosed is an apparatus which performs extraction of nucleic acid from a sample, amplification of the nucleic acid and detection of the nucleic acid in a continuous manner, wherein the nucleic acid reaction solution is hermetically sealed with a composition for preventing evaporation of the reaction solution at the steps of amplification and detection and wherein it possible to optically detect amplification of the nucleic acid through the composition.

Disclosed is an apparatus which performs extraction of nucleic acid from a sample, amplification of the nucleic acid and detection of the nucleic acid in a continuous manner, wherein the nucleic acid reaction solution is hermetically sealed with a composition for preventing evaporation of the reaction solution at the steps of amplification and detection and wherein it is possible to prevent leakage and scattering of the reaction solution by solidifying the composition after completion of the reaction.

The apparatus of the present invention is an apparatus capable of preventing evaporation of the reaction solution with any one of the compositions described in (1) to (3) above for preventing evaporation of reaction solutions.

Further, the apparatus of the present invention is an apparatus which uses a combination of a reaction vessel and a composition for preventing evaporation of a reaction solution, the combination being any one of the combinations described in (1a) to (1c) above.

Further, the present invention provides an apparatus which performs extraction of nucleic acid from a sample, amplification of the nucleic acid and detection of the nucleic acid in a continuous manner, the apparatus being capable of containing the prepackaged reagent described above.

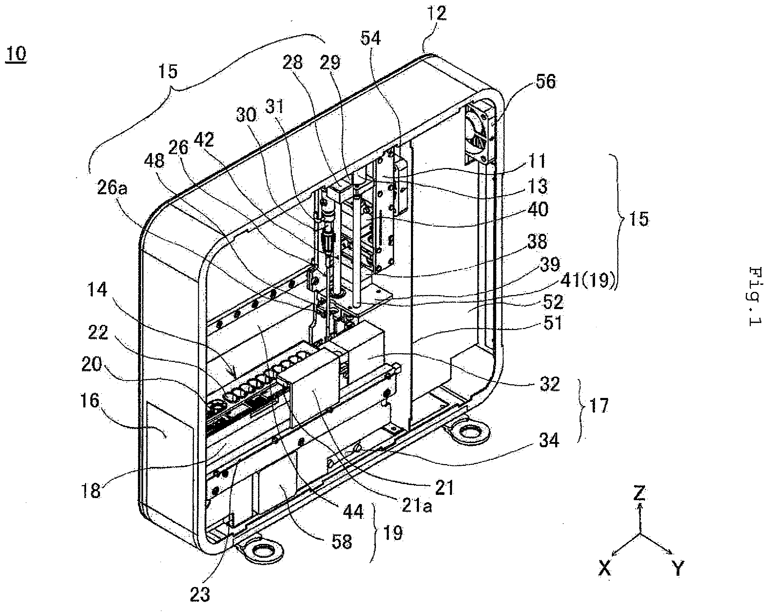

Next, an apparatus 10 that performs extraction of nucleic acid from a sample, amplification of the nucleic acid and detection of the nucleic acid in a continuous manner (hereinafter, referred to as "the first embodiment of a specimen testing device") will be described based on FIGS. 1 to 6.

The specimen testing device 10 is surrounded by a book-shaped housing 12 of, for example, 250 to 400 mm long (X axis direction), 70 to 100 mm wide (Y axis direction) and 300 to 500 mm high (Z axis direction). The housing 12 has: a test cartridge container 14 in which a plurality of (ten with this example) wells 22 which accommodate or can accommodate a specimen and one, two or more reagent solutions used to test the specimen, and a tip accommodation part 20 which accommodates a plurality of types (three types with this example) tips of testing tools are aligned in one row and provided, which displays specimen information for identifying or managing the specimen and test information showing test content on a seal 24 of a visible recording medium, and which is formed with a translucent member; an automatic testing unit (15 and 19) which causes a reaction of the specimen and the reagents accommodated in the test cartridge container 14 to obtain luminescence in a predetermined optical state; an optical measurement unit 17 which measures the luminescence produced as a result of the test in the automatic testing unit; a digital camera 28 which captures an image of content displayed on the test cartridge container 14 including the specimen information and test information to obtain image data; a thermal transfer printer mechanism 21 which can print a test result on blank spaces of the seal 24 of the test cartridge container 14; and a board 52 which has an integrated circuit such as a CPU for controlling the automatic testing unit (15 and 19), the optical measurement unit 17, the digital camera 28 and the thermal transfer printer mechanism 21.

The test cartridge container 14 is detachably loaded to a loading box 18 which is jointed with a fitting plate 16, the fitting plate 16 is provided to be manually drawn forth to the outside of the housing 12 from the housing 12.

A chamber in which the automatic testing unit (15 and 19), test cartridge container 14 and optical measurement unit 17 are provided, and a chamber in which the board 52 is provided are partitioned by a partitioning plate 51 to prevent destruction and contamination of a circuit due to droplets of a liquid which are sucked and discharged. A ventilation fan 54 is provided to penetrate the partitioning plate 51, and another ventilation fan 56 is provided to penetrate the housing 12 of the chamber in which the board 52 is provided.

The automatic testing unit (15 and 19) has a nozzle head 15 of a dispenser, and a moving mechanism 19 which can move the nozzle head 15 with respect to the test cartridge container 14 accommodated in the housing 12.

The nozzle head 15 of the dispenser has: a X axis moving body 11 which can move in the X axis direction corresponding to a longitudinal direction with respect to the test cartridge container 14 accommodated in the housing 12 by means of the moving mechanism 19; and a Z axis moving body 13 which is movably provided to be guided by a guide column 41 in up and down directions with respect to the X axis moving body 11. To the X axis moving body 11, a nut part jointed to the Z axis moving body 13 is screwed and a Z axis moving ball screw 43 described later which moves the Z axis moving body 13 in the up and down directions is rotatably attached, and the guide column 41 and a support plate 39 which is attached through the guide column 41 are attached.

The nozzle head 15 has: a nozzle 30 which is attached to the Z axis moving body 13, in communication with a cylinder which sucks and discharges gas through an air rubber tube 31 which is provided to project from a lateral face; a motor 40 which drives a piston in the cylinder; and a ball screw 42 which is rotatably attached.

Further, the support plate 39 which is attached to the X axis moving body 11 rotatably supports the ball screw 42 and, beneath the support plate 39, supports movably in front and back directions a tip detaching plate 48 in which a U-shaped hole greater than the diameter of the nozzle 30 and smaller than the outer diameter of the thickest portion of the tip is formed to detach a tip such as a carrier sealing tip 26 from the nozzle 30 and, on the upper side of the support plate 39, a motor 38 which drives the tip detaching plate 48 in the front and back directions is attached to the X axis moving body 11.

The digital camera 28 is attached to the X axis moving body 11 through a camera support plate 29, and captures an image by moving the nozzle head 15 to a position at which the digital camera 28 can capture the entire specimen information and test information on the seal 24 of the test cartridge container 14 accommodated in the housing 12.

The moving mechanism 19 which moves the nozzle head 15 of the dispenser with respect to the test cartridge container 14 accommodated in the housing 12 has: a rail 44 which engages with and guides the X axis moving body 11 of the nozzle head 15 in the longitudinal direction, that is, the X axis direction of the cartridge container 14; a X axis moving motor 58 which moves the nozzle head 15 along the X axis direction; the guide column 41 which guides the Z axis moving body 13 in the up and down directions, that is, the Z axis direction; the Z axis moving ball screw 43; and a Z axis moving motor. In addition, the cylinder, the ball screw 42 and the motor 40 correspond to an suction/discharging mechanism. Further, the guide column 41, the Z axis moving ball screw 43 and the Z axis moving motor correspond to the Z axis moving mechanism in the moving mechanism 19.

The optical measurement unit 17 has: a tip inserting unit 34; and a photoelectric unit 32 which has at least one photoelectric element such as a photoelectric multiplier tube which converts received luminescence into a predetermined electric signal.

The thermal transfer printer mechanism 21 is connected with the optical measurement unit 17 through the board 52, receives an electric signal matching the measurement result of the optical measurement unit 17 and performs printing on the seal 24 of the test cartridge container 14. The thermal transfer printer mechanism 21 is preferably provided such that, when the test cartridge container 14 is inserted in the housing 12, the thermal transfer printer mechanism 21 is positioned above without contacting the test cartridge container 14, accommodates the test cartridge container 14 and is lowered by, for example, a cam mechanism, and a printer head 21a of the thermal transfer printer mechanism 21 is positioned in a predetermined blank portion on the seal 24 of the test cartridge container 14. The printer head 21a is directed to automatically writing digital numbers on the seal 24 formed with a heat sensitive medium by forming digital numbers of predetermined digits and heating a predetermined segment of the digital numbers of the printer head 21a.

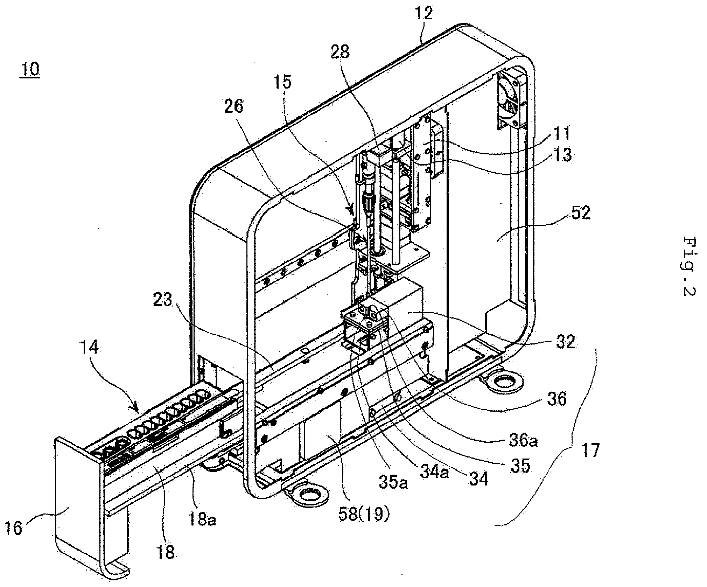

FIG. 2 illustrates a state where the test cartridge container 14 of the specimen testing device 10 is manually drawn forth from the housing 12. In addition, the thermal transfer printer mechanism 21 is removed for ease of description.

With the loading box 18 in which the test cartridge container 14 is loaded, a guide member 18a extending along the longitudinal direction of the loading box 18, that is, the X axis direction is provided to be guided by a guide rail 23 laid in the housing 12 along the X axis direction and manually moved in the X axis direction, so that it is possible to completely accommodate the test cartridge container 14 in the housing 12.

In addition, it is preferable to interlock insertion of the container 14 and upward and downward movement of the thermal printer mechanism 21 by providing the cam mechanisms in the guide member 18a and thermal transfer printer mechanism 21.

Further, a carrier sealing tip 26 in which particles 26c which are a plurality of carriers are accommodated is detachably attached to the nozzle 30 of the nozzle head 15.

The optical measurement unit 17 further has: a measurement block 36 at the rim of which a semi-circular hole 36a is formed and which is fixed to the photoelectric unit 32; and a measuring plate 35 at the rim of which an elongate hole 35a is formed below the measurement block 36 and above the tip insertion unit 34 and which is provided to be retreated back and forth along the longitudinal direction (X axis direction) of the elongate hole 35a by an electric magnet. The tip insertion unit 34 which is provided below the measurement plate 35 is formed in a box shape so that it enables a small diameter tube 26a of the carrier sealing tip 26 which is lowered passing through a cavity portion combined by the semi-circular hole 36a and elongate hole 35a to be inserted through a square hole 34a of the tip insertion unit 34. The measurement plate 35 and measurement block 36, and the photoelectric unit 32 are fixed to the housing 12 upon measurement, and scan and measure a plurality of particles 26c by raising and lowering the carrier sealing tip 26 with respect to the housing 12.

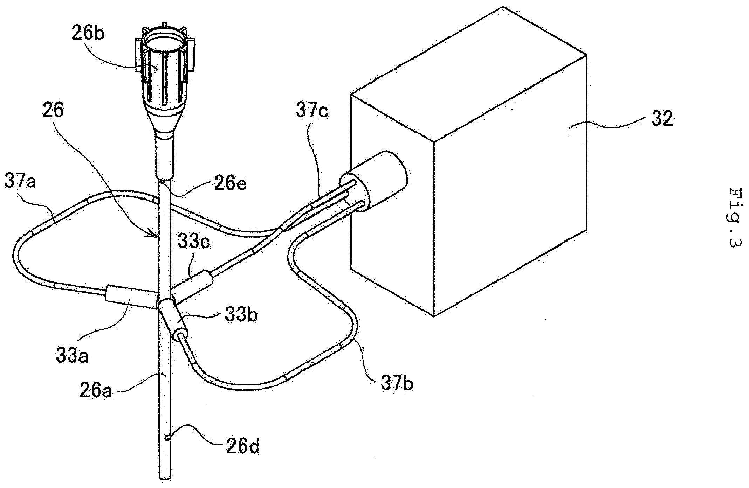

FIG. 3 is an optical system built in the optical measurement unit 17. The optical system is a device which is suitable to measure, for example, chemiluminescence, and has: three sets of optical fibers 37a, 37b and 37c; and light receiving ends 33, 33b and 33c provided at the front ends of the optical fibers and made of lenses. The light receiving ends 33a and 33b are arranged along a sidewall of the elongate hole 35a of the measurement plate 35, the light receiving end 33c is arranged in the sidewall of the semi-circular hole 36a of the measurement block 36, and these light receiving ends 33a, 33b and 33c surround the small diameter tube 26a of the carrier sealing tip 26 from three directions in a radial pattern. Upon insertion of the carrier sealing tip 26, the horizontal cross-sectional area of the cavity portion formed by the elongate hole 35a and semi-circular hole 36a is expanded by moving in a forward direction the measurement plate 35 using a magnetic force of the electric magnet, and, upon measurement, the horizontal cross-sectional area is narrowed by moving the measurement plate 35 in a backward direction and placing the measurement plate 35 close to the carrier sealing tip 26 inserted in the elongate hole 35a.

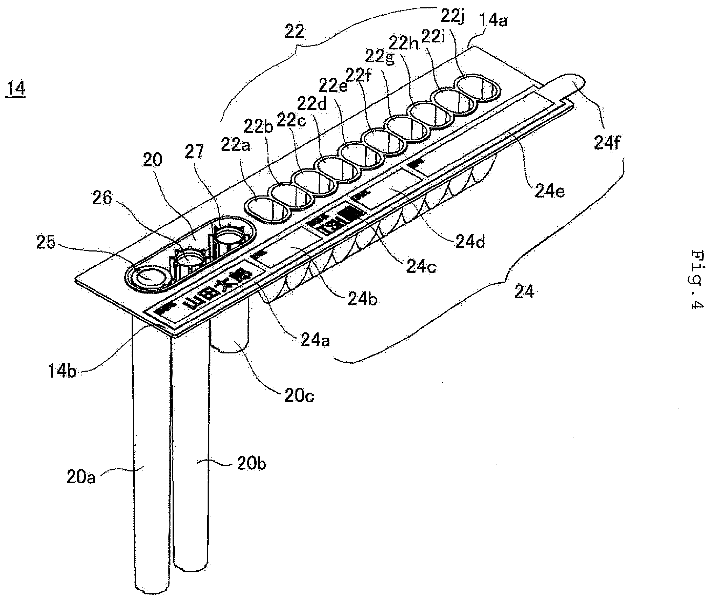

FIG. 4 is a view enlarging the test cartridge container 14.

A base plate 14a of the test cartridge container 14 has an opening part of the tip accommodation part 20 and opening parts of the well 22. The volume of each well 22 is, for example, about 1 cc to several cc, and, for example, 2 cc. In the tip accommodation part 20, three tips with this example, that is, a dispenser tip 25, the carrier sealing tip 26 and a piercing tip 27 are accommodated in cylindrical bodies 20a, 20b and 20c having the corresponding depths with the attachment opening parts directed upward such that the dispenser tip 25, the carrier sealing tip 26 and the piercing tip 27 are attached when the nozzle 30 is lowered and inserted. In the ten wells 22, a specimen and one, two or more reagent solutions used to test the specimen are accommodated, and the opening parts are blocked by one film which can be pierced by the piercing tip 27. In addition, the opening part of the tip accommodation part 20 is blocked by the seal which can be manually peeled off, and are used by peeling off the seal upon use. The test cartridge container 14 can be supplied to users as a prepackaged reagent which accommodates one or more reagent solutions that are used to test the sample (and which contain the composition for preventing evaporation of a reaction solution during nucleic amplification reaction.) The prepackaged reagent may include a tip such as the carrier sealing tip 26 (which is one embodiment of the reaction container as referred to in the present invention.)

In a seal pasting area 14b which is the medium attaching part of the base plate 14a of the test cartridge container 14, the seal 24 which visibly displays specimen information (24a and 24b) and test information (24c, 24d and 24e) showing test content is detachably pasted. Meanwhile, for the test information (24a and 24b), for example, a space 24a in which the name of a patient is hand-written and displayed and a space 24b in which an identification number of the patient is displayed are provided, and, for test information (24c, 24d and 24e), a space 24c in which a test item is displayed, a LOT number space 24d in which a LOT number indicating management information such as a manufacturing place, a manufacturing period, expiration date, the number of manufactured reagents, storage location and quality of one, two or more reagents accommodated in advance in the test cartridge container 14, and a remarks space 24e in which a test result measured by the optical measurement unit 17 is written and displayed are provided. The test items include, for example, TSH (thyroid stimulation hormone), in-vivo inflammation and allergy tests, and are displayed by, for example, two-dimensional codes as illustrated in FIG. 3. In addition, 24f denotes a pick-up part for peeling off the seal 24 from the base plate 14a.

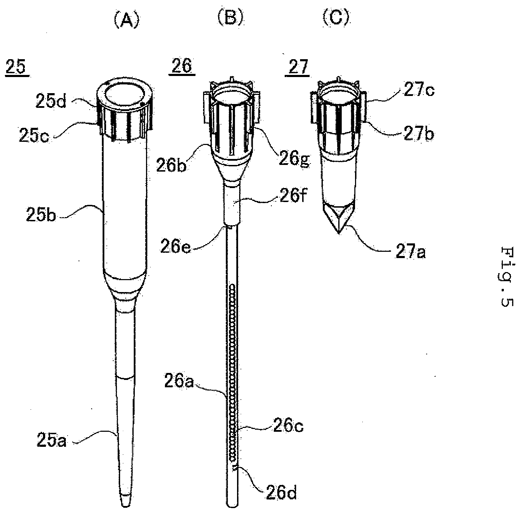

FIG. 5 illustrates three types of tips (25, 26 and 27) accommodated in the tip accommodation part 20 of the test cartridge container 14.

As illustrated in FIG. 5(A), the dispenser tip 25 is used to suck a liquid to accommodate the liquid in a tip, discharge a liquid moved between the wells 22 and accommodated, and transport the liquid between the wells 22. The dispenser tip 25 has: a small diameter tube 25a which has the thickness which allows the front end to be inserted into the well 22; a large diameter tube 25b which communicates with the small diameter tube 25a and has at a rear end an attachment opening part to which the nozzle 30 can be attached; and a plurality of elongated protrusions 25d provided in parallel to the axial direction, at the rear end part of the large diameter tube 25b.

As illustrated in FIG. 5(B), with the carrier sealing tip 26, the particles 26c which are a plurality of (fourth three with this example) carriers are aligned in one row in the small diameter tube 26a having the thickness which can be inserted into the well 22, and each particle is fixed with binding substances to which target substances marked by fluorescence can be bound, and is sealed inside by calking the small diameter tube 26a at positions 26d and 26e. The small diameter tube 26a communicates with the large diameter tube 26b through a filter unit 26 provided with a filter which allows only air to pass, and the opening part of the large diameter tube 26b is provided to be attached to the nozzle 30. In the surrounding of the large diameter tube 26b, a plurality of elongated protrusions 26g are provided in parallel to the axial direction.

As illustrated in FIG. 5(C), the piercing tip 27 has a sharp front end part 27a for piercing the film which blocks the opening part of the well 22 of the test cartridge container 14, the opening part of a rear end part 27b is attachable to the nozzle 30 and, in the outer periphery of the rear end part 27b, a plurality of elongated protrusions 27c are provided in parallel to the axial direction. In addition, with these tips, the length of the small diameter tube or front end part is, for example, 1 cm to 10 cm, the length of the large diameter tube is, for example, 1 cm to 10 cm and the diameter of the particle is, for example, 0.1 mm to 3 mm. Hence, the inner diameter of the small diameter tube 26a has the size which can hold this particle in one row, and is, for example, about 0.2 mm to 6 mm.

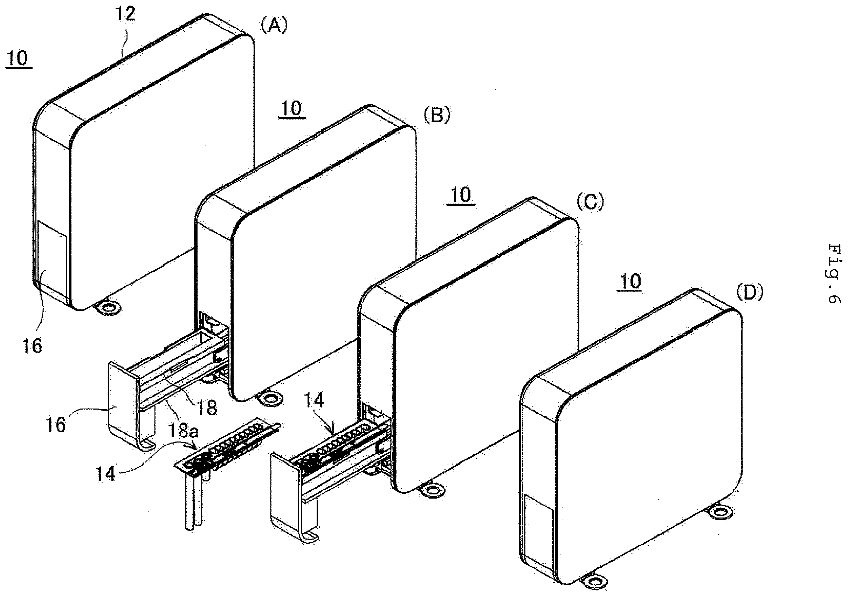

Then, the operation of the specimen testing device 10 according to the first embodiment will be described based on FIG. 6.

As illustrated in FIG. 6(A), in step S1, the fitting plate 16 of the housing 12 of the specimen testing device 10 is drawn forth by the hand. As illustrated in FIG. 6(B), in step S2, the loading box 18 is expanded to the outside of the housing 12. As illustrated in FIG. 6(C), in step S3, the test cartridge container 14 which accommodates a specimen of the test target, a test reagent and tips in advance is loaded in the loading box 18. In this case, in the seal 24 of the test cartridge container 14, the name of the patient belonging to the specimen information is hand-written, and test information showing test content is written in advance. As illustrated in FIG. 6(D), in step S4, the loading box 18 and loaded test cartridge container 14 are inserted and accommodated in the housing 12 by the hand.

In the state of FIG. 6(D), the following processing is performed.

In step S5, the nozzle head 15 is moved to the tip accommodation part 20 of the test cartridge container 14 to place the nozzle 30 above the piercing tip 27. The nozzle 30 is lowered along the Z axis direction to insert, push in and attach the front end of the nozzle 30 to the opening part of the piercing tip 27.

In step S6, the nozzle 30 to which the piercing tip 27 is attached is positioned sequentially above each well 22 of the test cartridge container 14, and then is lowered to pierce the film which covers the ten wells 22.

In step S7, when all wells 22 are pierced, the nozzle 30 moves to the position at which the piercing tip 27 of the tip accommodation part 20 is accommodated, a U-shaped groove of the tip detaching plate 48 is placed close to the nozzle 30 and the nozzle 30 is moved along an upper direction (Z axis direction) to attach and detach the piercing tip 27 to and from the inside of the cylindrical body 20c of the tip accommodation part 20.

In step S8, the nozzle 30 is moved above the position at which the dispenser tip 25 (or carrier sealing tip 26) of the tip accommodation part 20 is accommodated and is lowered along the Z axis direction, and the front end of the nozzle 30 is inserted, pushed in and attached to the opening part of the dispenser tip 25 (or the carrier sealing tip 26).

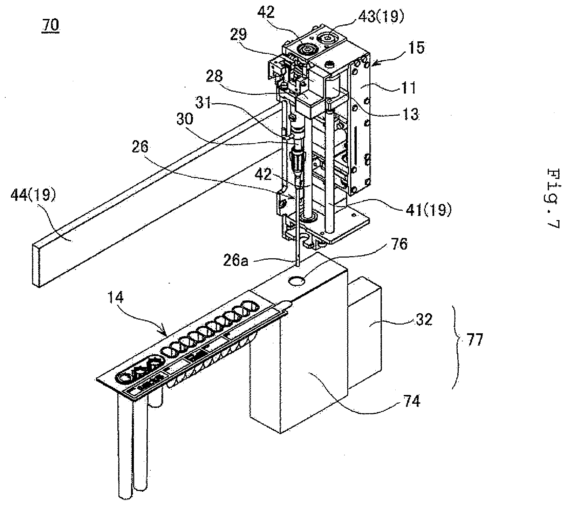

Next, FIG. 7 illustrates a specimen testing device 70 according to a second embodiment.

The specimen testing device 70 differs in using an optical measurement unit 77 instead of the optical measurement unit 17 used in the specimen testing device 10 according to the first embodiment.

The optical measurement unit 77 has: the photoelectric unit 32 which has at least one photoelectric element; and a scanning/measuring unit 74 which has a hole 76 in which the small diameter tube 26a of the carrier sealing tip 26 can be inserted, and in which each of the light receiving ends 33a, 33b and 33c of the optical fibers 37a, 37b and 37c provided to surround the small diameter tube 26a of the carrier sealing tip 26 inserted through the hole 76 and connected with the photoelectric unit 32 is provided to move along the axial direction of the small diameter tube 26a inserted through the hole 76. That is, the optical measurement unit 77 differs from the optical measurement unit 17 according to the first embodiment in that each of the light receiving ends 33a, 33b and 33c is not fixed to the housing 12 upon measurement, and is relatively movable.

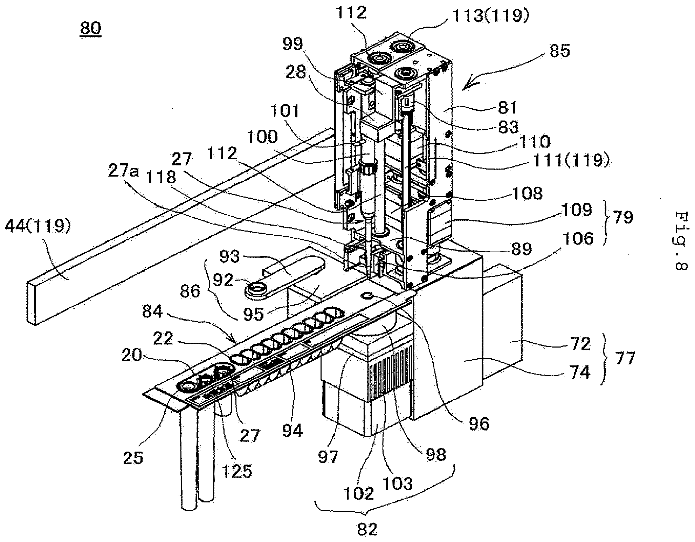

FIG. 8 illustrates a specimen testing device 80 according to a third embodiment.

The specimen testing device 80 differs from the specimen testing devices 10 and 70 according to the first and second embodiments in mainly having: a magnetic member 79 which has a magnet 106 provided to contact and separate from the small diameter tube 25 to apply and remove the magnetic force to and from the small diameter tube 25a of the dispenser tip 25; a temperature controller 82 which controls the temperature of a well 96 provided in a test cartridge container 84; and a cap moving mechanism 86 which blocks the well 96 by means of a cap 92. The well 96 is one embodiment of the reaction container as referred to in the present invention. The test cartridge container 84 can be supplied to users as a prepackaged reagent which accommodates one or more reagent solutions that are used to test the sample (and which contain the composition for preventing evaporation of a reaction solution during nucleic amplification reaction.)

The specimen testing device 80 is mounted in the housing 12 similar to the specimen testing devices 10 and 70 according to the first and second embodiments. The housing 12 has: a test cartridge container 84 in which a tip accommodation part 20 which accommodates a plurality of types (three types including two types of dispenser tips 25 and 125 having different volumes and piercing tip 27 with this example) of tips, a plurality of (ten with this example) wells 22 which accommodate or can accommodate a specimen and one, two or more reagent solutions, and the well 96 which is provided spaced apart from the well 22 and of which temperature is controlled are aligned in one row and provided, which displays specimen information for identifying or managing the specimen and test information showing test content on a seal 94 of a visible recording medium, and which is formed with a translucent member; an automatic testing unit (85 and 19) which causes a reaction of the specimen and the reagents accommodated in the test cartridge container 84 to obtain predetermined luminescence; an optical measurement unit 177 which measures the luminescence produced as a result of the test in the automatic testing unit; a digital camera 28 which captures an image of content displayed on the test cartridge container 84 including the specimen information and test information to obtain image data; a thermal transfer printer mechanism 21 (see FIG. 1) which can print a test result on blank spaces of the seal 94 of the test cartridge container 84 as a writing mechanism; and the magnetic member 79; the temperature controller 82; the cap moving mechanism 86; and a board 52 which has an integrated circuit such as a CPU for controlling the automatic testing unit (85 and 19), optical measurement unit 177, digital camera 28, thermal transfer printer mechanism 21, magnetic member 79, temperature controller 82 and cap moving mechanism 86.

The test cartridge container 84 is provided to be manually drawn forth from the housing 12 to the outside of the housing 12 as illustrated in FIGS. 1 and 2. In addition, the volume of the well 96 which controls the temperature of the test cartridge container 84 is, for example, 0.2 cc.

The automatic testing unit (85 and 19) has: a nozzle head 85 of a dispenser; and a moving mechanism 119 which can move the nozzle head 85 with respect to the test cartridge container 84 accommodated in the housing 12.

The nozzle head 85 of the dispenser has: a X axis moving body 81 which can move in the X axis direction corresponding to a longitudinal direction with respect to the test cartridge container 84 accommodated in the housing 12 by means of the moving mechanism 119; and a Z axis moving body 83 which is provided to be guided by a guide column 111 in up and down directions with respect to the X axis moving body 81 and moved. To the X axis moving body 81, a nut part jointed to the Z axis moving body 83 is screwed and a Z axis moving ball screw 113 described later which moves the Z axis moving body 83 in the up and down directions is rotatably attached, and the guide column 111 and a support plate 89 which is attached through the guide column 111 are attached.

The nozzle head 85 has: the nozzle 100 which is attached to the Z axis moving body 83, and in communication with a cylinder which sucks and discharges gas through an air rubber tube 101 which is provided to project from a lateral face; a motor 110 which drives a piston in the cylinder; and a ball screw 112 which is rotatably attached.

Further, the support plate 89 which is attached to the X axis moving body 81 rotatably supports the ball screw 113 and, beneath the support plate 89, supports movably in front and back directions a tip detaching plate 118 in which a U-shaped hole greater than the diameter of the nozzle 100 and smaller than the outer diameter of the thickest portion of the tip is formed to attach and detach a tip such as the dispenser tip 25 to and from the nozzle 100 and the magnet 106 which is provided to contact and separate from the small diameter tube 25 a of the dispenser tip 25 attached to the nozzle 100 and which can apply and remove the magnetic force to and from the interior of the small diameter tube 25a from an outside, and, on the upper side of the support plate 89, a motor 108 which drives the tip detaching plate 118 and a motor 109 which drives the magnet 106 are attached to the X axis moving body 81. The magnet 106 and motor 109 correspond to the magnetic member 79.

The digital camera 28 is attached to the X axis moving body 81 through a camera support plate 99, and captures an image by moving the nozzle head 85 to a position at which the digital camera 28 can capture the entire specimen information and test information on the seal 94 of the test cartridge container 84 accommodated in the housing 12.

The moving mechanism 119 which moves the nozzle head 85 of the dispenser with respect to the test cartridge container 84 accommodated in the housing 12 has: a rail 44 which engages with and guides the X axis moving body 81 of the nozzle head 85 in the longitudinal direction, that is, the X axis direction of the cartridge container 84; a X axis moving motor 58 (see FIG. 1) which moves the nozzle head 85 along the X axis direction; the guide column 111 which guides the X axis moving body 83 in the up and down directions, that is, the Z axis direction; the Z axis moving ball screw 113; and a Z axis moving motor. In addition, the ball screw 112 and motor 110 correspond to an suction/discharging mechanism. Further, the guide column 111, the Z axis moving ball screw 113 and the Z axis moving motor correspond to the Z axis moving mechanism in the moving mechanism.

In addition, the specimen testing device 80 according to the present embodiment also has the thermal transfer printer mechanism 21 which is a writing mechanism. The thermal transfer printer mechanism 21 is as described above.

The cap moving mechanism 86 has: a cap 92 which covers the opening part of the well 96; an arm 93 in which the cap 92 is provided at one end and the other end is axially supported by a rotary shaft to rotate 90 degrees by a rotary shaft; and a rotation driving unit 95 which has a motor driving the rotary shaft.

Further, the specimen testing device 80 can further press, shake or move the cap 92 which blocks the opening part of the well 96 of the test cartridge container 84, using the nozzle 100 which can be pressed, shaken or moved by the moving mechanism 119 including the Z axis moving mechanism along the Z axis direction, X axis direction and Y axis direction. That is, the nozzle 100 which is driven by the moving mechanism 119 including the Z axis moving mechanism corresponds to a cap-blocked-duration functioning mechanism. In this case, the cap 92 is preferably biased and supported by the elastic force with respect to the rotary shaft in the Z axis direction.

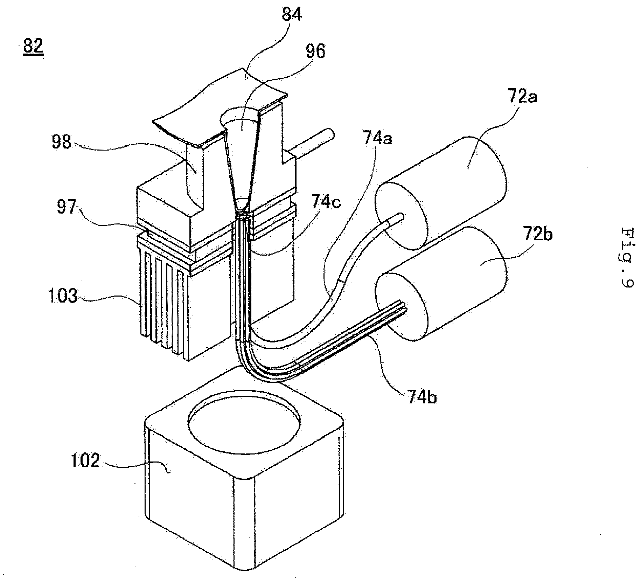

As illustrated in FIG. 9, the temperature controller 82 has: a temperature control block 98 in which a tapered fitting hole having the shape and size fitting with the well 96 of the test cartridge container 84 which is the well accommodation hole is bored and provided in the center; a peltier element unit 97 which has a peltier element which is provided in contact with the temperature control block 98 and which is a heating/cooling unit; a fin 103 which is provided below the peltier element unit 97; and a fin accommodation frame body 102 which is provided below the fin 103, and a radiation optical fiber 74a and six light receiving optical fibers 74b extending from the bottom of the fitting hole, passing the fin 103 through the peltier element part 97 and one end of the radiation optical fiber 74a are connected with an excitation light source 75b, one end of the light receiving optical fiber 74b is connected with the photoelectron multiplying tube 72b, and the other ends 74c of these optical fibers 74a and 74b are bundled around the radiation optical fiber and provided such that the front ends are positioned in the bottom of the fitting hole which is the well accommodation hole.

Meanwhile, the optical fibers 74a and 74b pass a fiber accommodation part 174 of the optical measurement unit 177, and are connected with the excitation light source 72a and photoelectron multiplier tube 72b built in the photoelectric/light source unit 72.

Next, the operation of the specimen testing device 80 according to the third embodiment will be described.

Steps are the same as step S1 to step S8 except that the nozzle head 85 is used instead of the nozzle head 15, the nozzle 100 is used instead of the nozzle 30 and the test cartridge container 84 is used instead of the test cartridge container 14.

In the state of FIG. 6(D), the following processing is performed.

Hereinafter, an operation of controlling the temperature of DNA or genome and performing PCR processing instead of conducting an allergy test described in the first embodiment will be described.

In the well 22a of the test cartridge container 84, for example, a specimen such as a mucous membrane of the mouth collected from the test subject is accommodated. In the well 22b, a genome extraction reagent is accommodated.

In the well 22c, a magnetic particle suspension is accommodated. In the well 22d, a separate solution is accommodated. The well 22e is empty. In the well 22f to well 22i a primer containing solution which is a PCR reagent and rinse liquid are accommodated. In the well 22j, mineral oil is accommodated, which is an example of the composition for preventing evaporation of a reaction solution during nucleic amplification reaction. Further, the tip accommodation part 20 accommodates the two types of dispenser tips 25 and 125 and piercing tip 27.

In step S9, the nozzle 100 is moved to the position of the dispenser tip 25 accommodated at the end of the tip accommodation part 20, and is lowered to be attached to the nozzle 100 to extract the genome, and the dispenser tip 25 is moved to the well 22b by the moving mechanism 119 to suck a corresponding extraction reagent using the suction/discharging mechanism. The dispenser tip 25 is moved to the well 22a which accommodates the specimen, and discharges in the well 22a the liquid sucked in the dispenser tip 25. Further, the dispenser tip 25 is moved to the well 22c to suck the magnetic particle suspension, and is moved to the well 22a to discharge the magnetic particle suspension, and, if there are reagents which are necessary to perform extraction, the reagents are transported to the well 22a using the dispenser tip 25 and discharged. These mixed liquids accommodated in the well 22a are repeatedly sucked and discharged to be reacted while being stirred and incubated, and the extracted DNA is bound to the surfaces of the magnetic particles and is caught.

In step S10, the magnet 106 is placed close to the small diameter tube 25a of the dispenser tip 25 using the magnetic member 79 to produce the magnetic field therein, and the magnetic particles are attracted to the inner wall of the small diameter tube 25a to separate DNA.

In step S11, the dispenser tip 25 for genome extraction is moved by the moving mechanism 119 while the magnetic particles catching the DNA are attracted to the inner wall, and is positioned over the well 22d which accommodates the separate solution, and the front end outlet part of the dispenser tip 25 is inserted in the well 22d and repeats sucking and discharging the separate solution with the magnetic particles attracted to the inner wall to separate the DNA from the magnetic particles. The DNA solution containing the DNA separated from the magnetic particles is discharged into and accommodated in the empty well 22e, and the dispenser tip 25 for genome extraction is transported to the original accommodation position in the tip accommodation part 20 while the magnetic particles are attracted to the inner wall to attach and detach using the tip detaching plate 118.

In step S12, the nozzle head 85 is moved, the nozzle 100 of the nozzle head 85 is moved to a new dispenser tip 125 for PCR accommodated at the middle position in the tip accommodation part 20, and the nozzle 100 is lowered by the Z axis moving mechanism to insert and attach the nozzle 100 in and to the attachment opening part of the accommodated dispense tip 125 for PCR.

In step S13, the nozzle head 85 is moved, and the arm 93 is rotated 90 degrees as illustrated in FIG. 9 to open the cap 92 and expose the opening part of the well 96 to the outside. Next, using the dispenser tip 125 for PCR, reagents for PCR accommodated in the well 22f to well 22i such as a primer containing solution labeled by a fluorescent material is sucked, dispensed and accommodated in the well 96. The above process is repeated until dispensing of the required reagents is finished.

In step S 14, the dispenser tip 125 is rinsed, and then the nozzle head 85 is moved to suck the extracted DNA liquid accommodated in the well 22e to dispense in the well 96. Then, the dispenser tip 125 is used and moved to the well 22j, and sucks the mineral oil and discharges the mineral oil (which is an example of the composition for preventing evaporation of a reaction solution during nucleic amplification reaction) in the well 96 to introduce.

In step S15, the cap 92 is rotated 90 degrees to cover the opening part of the well 96.

In step S16, the nozzle 100 is lowered to press the cap 92 using the Z axis moving mechanism.

In step S17, the temperature controller 82 controls the temperature of the well 96 according to a PCR method. The temperature control according to the PCR method is directed to setting the temperature of the well 96 to 94°C to denature two strands of DNA of the administered specimen to a single strand, and set the temperature of the well 96 to 50°C to 60°C to anneal and hybridize the single strand of DNA and primer. Next, a cycle of an operation of performing incubation by synthesizing complementary DNA strands to a single strand and setting the temperature to 74°C is repeated a predetermined number of times, and temperature control is performed for about several minutes.

In this case, excitation light is radiated using the optical fibers 74a and 74b provided in the fitting hole which is the well accommodation hole of the temperature control block 98, and the fluorescence intensity to be produced is received by the optical fiber 74b and is converted into an electric signal by the photoelectron multiplier tube 72b to measure the fluorescence intensity.

In step S18, the measurement result is analyzed by the control unit of the board 52, is output to the thermal transfer printer mechanism 21, is printed as one item of the test information in the remarks space of the seal 24 by the printing head 21a and is displayed by numbers.

In step S19, the digital camera 28 captures an image of specimen information and test information on the seal 94 of the test cartridge container 84 as image data according to a command signal from the board 52. In this case, an analyzing unit of the control unit searches for data which can be analyzed, from the image data, when finding a two-dimensional barcode data showing the test content included in the test information, and analyzes the two-dimensional barcode data to obtain analyzed data, and the data synthesizing unit of the control unit synthesizes and stores the analyzed data and image data in a memory as data which can be output.

In step S20, the dispenser tip 125 attached to the nozzle 100 moves to the tip accommodation part 20, is moved directly above the position at which the dispenser tip 125 is accommodated, and places the U-shaped groove of the tip detaching plate 118 close to the nozzle 100, and the nozzle 100 is moved in the upper direction to attach and detach the dispenser tip 125 to and from the inside of the cylindrical body 20b of the tip accommodation part 20.

In step S21, when testing of the specimen is finished, the loading box 18 in which the test cartridge container 84 is loaded is manually drawn forth from the housing 12, the seal 94 pasted on the test cartridge container 84 is peeled off and is stuck to a mat board for management which is additionally prepared and stored, and a new test cartridge container 84 is further loaded to the housing 12 while the test cartridge container 84 is discarded, so that it is possible to test a new specimen. According to the present embodiment, the cap 92 can be pushed using the moving mechanism, so that it is possible to reliably block the opening part of the well 96 and easily prevent dew condensation and release the cap 92.

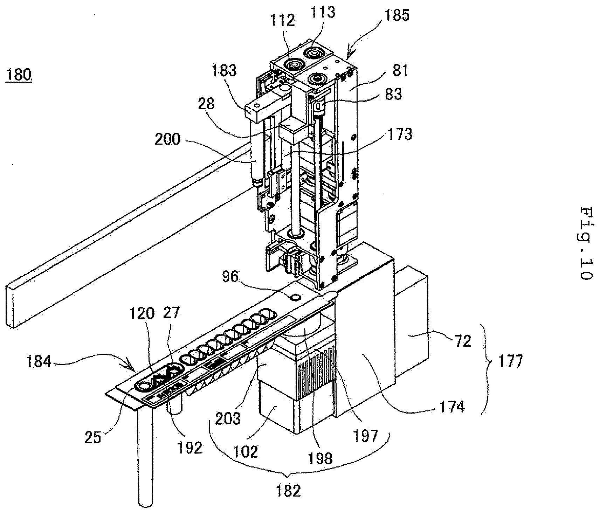

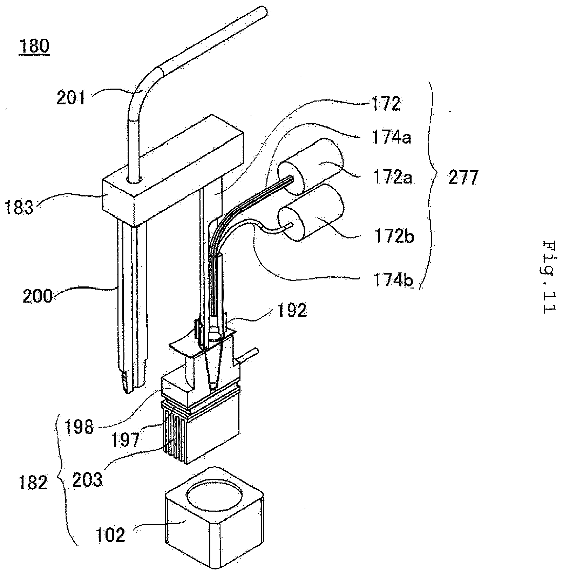

FIGS. 10 and 11 illustrate a specimen testing device 180 according to a fourth embodiment.

In addition, the same components as in the specimen testing device 80 illustrated in FIG. 8 will be assigned the same reference numerals or will not be described without assigning the reference numerals.

The specimen testing device 180 differs from the specimen testing device 80 according to the third embodiment illustrated in FIG. 8 in that the nozzle head 185 has: a nozzle 200 to which the dispenser tip 25 in communication with the cylinder which sucks and discharges gas through an air rubber tube 201 are attachable; a nozzle support body 183 which interlocks with the Z axis moving body 83 which can move in the Z axis direction, and to which the nozzle 200 is attached; and a measurement rod 172 (see FIG. 11) in which the end of the light receiving optical fiber 174a and the end of the radiation optical fiber 174b are provided to measure luminescence from above a translucent cap 192 which covers the opening part of the well 96 of the test cartridge container 184 attached to the nozzle support body 183.

Additionally, the specimen testing device 180 differs from the specimen testing device 80 according to the third embodiment in that the cap moving mechanism 86 is not provided, and the cap 192 is accommodated in advance in the tip accommodation part 120 of the test cartridge container 184 in place of the carrier sealing tip 26, and is attached to the front end of the nozzle 200 or front end of the measurement rod 172 by lowering the nozzle 200 and the measurement rod 172 by the Z axis moving mechanism and is used upon pressing or upon measurement. Thus, the test cartridge container 184 also differs in that the cap 192 can be accommodated in the tip accommodation part 120.

Further, as illustrated in FIG. 11, an optical measurement unit 277 and the temperature controller 182 differ from the optical measurement unit 177 and temperature controller 82 according to the third embodiment.

With the optical measurement unit 277, the end of the light receiving optical fiber 174a and the end of the radiation optical fiber 174b are provided in the measurement rod 172, the other end of the light receiving optical fiber 174a is connected with the photoelectric element 172a and the other end of the radiation optical fiber 174b is connected with the light source unit 172b.

Further, the temperature controller 182 only has: a temperature control block 198 in which a tapered fitting hole having the shape and size fitting with the well 96 of the test cartridge container 184 is bored and provided in the center as the well accommodation hole; a peltier element unit 197 which has a peltier element which is provided in contact with the temperature control block 198 and which is a heating/cooling unit; and a fin 203 which is provided below the peltier element unit 197, and a fin accommodation frame body 102 which is provided below the fin 203 and the ends of optical fibers are not provided in the bottom of the fitting hole and the optical fibers do not pass the fin 203.

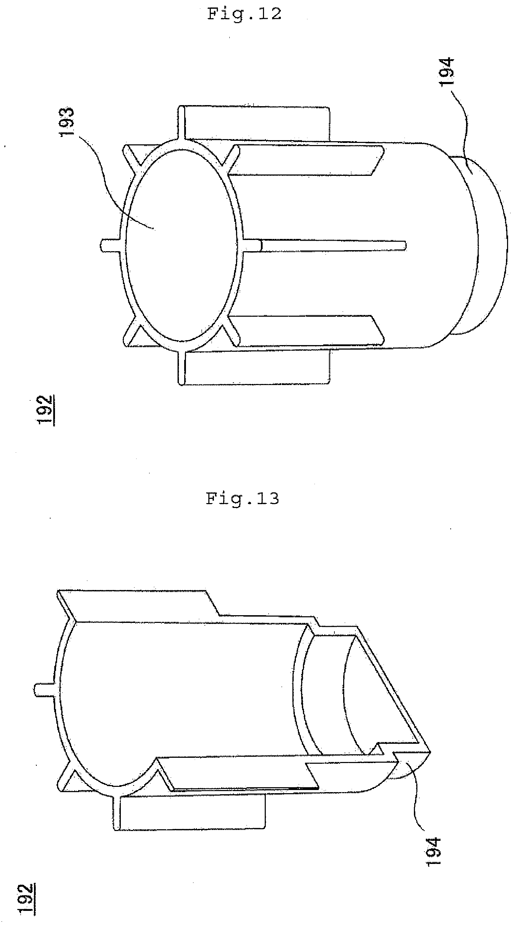

FIGS. 12 and 13 illustrate the cap 192. The cap 192 has: an attachment opening part 193 to which the measurement rod 172 and nozzle 200 can be attached; and the fitting part 194 which fits to the opening part of the well 96. With the device according to the present embodiment, the cap can block the opening part of the well 96 without providing the cap moving mechanism, so that it is possible to simplify the structure of the device. Further, if there is a concern that the cap contaminates a specimen, the cap can be accommodated in the test cartridge container and discarded together with a test cartridge container after the test is finished like a tip, so that it is possible to provide safe management.

Next, a specimen testing device according to a fifth embodiment will be described based on FIG. 14.

A specimen testing device 280 according to the present embodiment has: two test cartridges 284 which are provided in housings of, for example, about 250 to 400 mm long (X axis direction), 140 to 200 mm wide (Y axis direction) and 300 to 500 mm high (Z axis direction), in which tip accommodation parts 220a, 220b and 220c which accommodate a specimen and a plurality of types (three types with this example) of tips which are one, two or more testing tools used to test the specimen are aligned in a row, which displays specimen information for identifying or managing the specimen and test information showing test content on a seal 224 which is a visible recording medium, and which are aligned in parallel; two test cartridge containers 384 in which a well 322 which accommodates and can accommodate a specimen and one, two or more reagent solutions used to test the specimen and which is a plurality of (ten with this example) accommodation parts is provided in one row, which displays specimen information for identifying or managing the specimen and test information showing test content on a seal 324 which is a visible recording medium, and which are formed with translucent members and aligned in parallel; an automatic testing unit (285 and 289) which causes a reaction of the specimen and the reagents accommodated in the two test cartridge containers 384 to obtain a predetermined optical state (for example, luminescence); an optical measurement unit which measures the optical state produced as a result of the test in the automatic testing unit; a digital camera 228; a thermal transfer printer mechanism which can print a test result on blank spaces of the seals 224 and 324 of the test cartridge containers 284 and 384; and a board which has an integrated circuit such as a CPU for controlling the automatic testing unit (285 and 289), the optical measurement unit, the digital camera 228 and the thermal transfer printer mechanism. 285a individually denotes a unit which mainly has a Z axis moving mechanism which moves the nozzle 230 in the Z axis direction.

Meanwhile, with the two cartridge containers 284, dispenser tip 225, carrier sealing tip 226 and piercing tip 227 which are a plurality of types (three types with this example) of tips of the testing tools are accommodated or can be accommodated in each of the tip accommodation parts 220a, 220b and 220c. The dispenser tips 225 are already attached to the nozzle 230 of the nozzle head 285, and therefore the accommodation parts 220a are empty.

With the two cartridge containers 284, the opening parts of the tip accommodation parts 220a, 220b and 220c are provided in a base plates 284a. In the seal pasting area which is a medium attaching part of the base plate 284a, the seal 224 is detachably pasted which has a specimen information space 224a and a test information space 224b showing test content. Meanwhile, in the specimen information space 224a, a QR code is printed in advance and a space to be filled by hand writing is provided and, in the test information space 224b, test information is printed in advance and a space to be filled by hand writing or a blank space for printing is provided. Similarly, with the two cartridge containers 384, the base plates 384a have wells 322a to 322j which accommodate ten reagent solutions and specimen solutions. In the seal pasting area which is a medium attaching part of the base plate 384a, the seal 324 is detachably pasted which has a specimen information space 324a and a test information space 324b showing test content. Meanwhile, in the specimen information space 324a, a QR code is printed in advance and a space to be filled by hand writing is provided and, in the test information space 324b, test information is printed in advance and a space to be filled by hand writing or a blank space for printing is provided. The test cartridge container 384 can be supplied to users as a prepackaged reagent which accommodates in the base plate 384a one or more reagent solutions that are used to test the sample (and which contain the composition for preventing evaporation of a reaction solution during nucleic amplification reaction.) The prepackaged reagent may include a tip such as the carrier sealing tip 226 (which is one embodiment of the reaction container as referred to in the present invention.)

In addition, all cartridge containers 284 and 384 aligned in the specimen testing device have common content of test information when the cartridge containers 284 and 384 are used for the same test. Further, although the test cartridge containers 284 and 384 aligned in a row (along the X axis direction) have common specimen information, the test cartridge containers 284 and 384 in the other row support a different specimen, these have specimen information different from the above specimen information.

For the automatic testing unit (285 and 289), the two nozzles 230 and 230 are provided, and each nozzle 230 is detachably attached with the dispenser tip 225 and each dispenser tip 225 is provided to move along the cartridge containers 284 and 384 in two rows. In addition, 244a and 244b denote rails which move the nozzle head 285 in the X axis direction and belongs to the moving mechanism 289.

In addition, the digital camera 228 is provided to be rotated a certain angle by a rotating mechanism 228a having the rotary shaft along the X axis direction, so that one digital camera 228 alone can cover the test cartridge containers 284 and 384 in the two rows. Further, the optical measurement unit, thermal transfer printer mechanism and optical measurement unit are also provided to move in the Y axis direction, so that one of the optical measurement unit, thermal transfer printer mechanism or optical measurement unit alone can support the test cartridge containers in the two rows, thereby making the device scale compact. According to the present embodiment, a plurality of tests can be processed in parallel, so that it is possible to perform efficient and quick processing.

The above-described embodiments are specifically described for better understanding of the present invention, and by no means limit other embodiments. Consequently, the present invention can be changed within a range without changing the spirit of the invention. Although, for example, cases of DNA have been described with the above embodiments, the present invention is naturally applicable to other tests of other nucleic acids such as RNA. Further, the numerical values, the number of times, shape the number of items and amount used in the above description are by no means limited to the above cases.

Further, types of tips, a cap and rod which need to be accommodated as a configuration of the test cartridge container, the structure and the number of tips, cap and rod, the number of or volume of wells, and content of specimen information and test information are only examples, and these can be adequately changed according to a specimen and test content.

Further, the above components such as each nozzle head, each type of tips, each cap, each nozzle, each temperature controller, each optical measurement unit, each test cartridge container and magnetic members are appropriately deformed and can be combined at random.

For example, it is possible to use the carrier sealing tip and use the test cartridge container which has wells of which temperature are controlled, and the temperature controller. Further, the above reagent, specimen and processing process are only examples, and other reagents, specimens and processing processes can be naturally used.

Although only cases have been described where one row or two rows of test cartridge containers are loaded in the specimen testing device and used, the present invention is by no means limited to this case and the present invention is naturally applicable to three or more rows of test cartridge containers. Further, when two rows of test cartridge containers are loaded and used, the present invention is by no means limited to this case, the test cartridge containers used in the first embodiment may be naturally aligned, loaded and used.

### EXAMPLES

Hereinbelow, the present invention will be described more specifically with reference to the following Examples. However, the present invention is not limited to these Examples.

### [Example 1] Composition for Preventing Evaporation

### 1. Preparation of Oil for Preventing Evaporation (Low-Temperature Solidification Type)

A desired amount (0.5, 0.75, 1.00, 1.25 or 1.50 g) of solid paraffin (Wako special grade paraffin; mp: 44-46°C) melted by heating at 60-80°C was mixed with 10.00 g of liquid paraffin (Sigma special grade) to prepare an oil for preventing evaporation. The thus prepared oils for preventing evaporation were used as samples in the following experiments.

It was confirmed that the solid and the liquid paraffin had the following performance or properties.
- Water (50 µl) and the solid or liquid paraffin (50, 100 or 200 µl) were filled in a nucleic acid amplification reaction vessel, and the weight loss during nucleic acid amplification reaction was measured. The residual water weight was not lower then 99%±0.3%.
- Insoluble in water (perfect separation).
- Smaller liquid phase specific gravities than water.
- No inhibition of nucleic acid amplification reaction.
- Liquid phase (of either the solid or liquid paraffin) having a spectral transmittance (520 nm, 25°C) of 90% or more.
- No emission of fluorescence (especially around the wave-length of detection light).
- No need of special disposal.

### 2. Checking for the Melting (Solidification) of the Oil for Preventing Evaporation

### 2-1 Confirmation of Clarification (Clouding) by Visual Observation and Confirmation of Solidification by Touching with Spatula

The melting point (solidifying point) of the low-temperature solidification type oil for preventing evaporation that was prepared in section 1 above was quantitatively determined as described below.
- Water and five types of oils for preventing evaporation (A-1 to A-5) (20 µl for each) were added to PCR containers MicroAmp (Applied Biosystems) (hereinafter, referred to as sample containers).
- Each of the above-described sample containers was mounted on a heat block fitting its outer shape, followed by temperature adjustment on a thermostat (AS ONE Corporation).
- With a gradual decrease in temperature from 60°C by 5°C, the property of the sample in each temperature range was evaluated both by visual observation and by touching with a spatula.

The results are shown in Table 1. In this Table, S represents solid and L represents liquid. Oils for preventing evaporation (sample ID: A-1, A-2, A-3, A-4 and A5) did not become cloudy when they were in the liquid state. When they became cloudy, they had already turned into solids. Thus, the phase change from liquid to solid was sharp.

**Table 1**

| Sample ID | Liquid paraffin | Wako special grade paraffin | Wako special grade paraffin | Container: MicroAmp 8-well | | | | | | | |
|---|---|---|---|---|---|---|---|---|---|---|---|
| | | | | Thermal Cycle: 60degC:5min →30degC:1min →-5degC step (holding time 1min / each step) | | | | | | | |
| | Amount added (g) | Gross weight including container, before addition | Percent addition on weight basis (relative to liquid paraffin taken as unity) | | 5 | 10 | 15 | 20 | 25 | 30 | 60 |
| A-1 | 10.0000 | 0.5000 | 5.00% | 5.00% | S | L | L | L | L | L | L |
| A-2 | 10.0000 | 0.7500 | 7.50% | 7.50% | S | S | L | L | L | L | L |
| A-3 | 10.0000 | 1.0000 | 10.00% | 10.00% | S | S | S | L | L | L | L |
| A-4 | 10.0000 | 1.2500 | 12.50% | 12.50% | S | S | S | L | L | L | L |
| A-5 | 10.0000 | 1.5000 | 15.00% | 15.00% | S | S | S | S | L | L | L |

### [Example 2] Verification of the Effect of the Wetting Tension of Nucleic Acid Amplification Reaction Vessel upon Fluorescence Detection

The following experiments were performed in order to verify the effect upon fluorescence detection of the ability of mineral oil for preventing evaporation of a nucleic acid amplification reaction solution during amplification reaction to wet the inner surface of a nucleic acid amplification reaction vessel.

### 1. Preparation of Nucleic Acid Amplification Reaction Vessels

The inner surfaces of white-colored PCR tubes (Roche) were coated with a water- and oil-repellent agent (Fluoro Technology) in a film thickness of 1 µm or less to thereby reduce the wetting tension of the inner surfaces of the tubes. Untreated tubes were also prepared. Thus, two types of nucleic acid amplification reaction vessels with different wetting tensions on their inner surfaces were prepared. The ability of mineral oil (Applied Biosystems) to wet these two types of reaction vessels was evaluated according to the method specified in JIS K6768 with necessary modifications. As a result, it was confirmed that the inner surface of the treated reaction vessel repelled the mineral oil, whereas the mineral oil spread to wet the inner surface of the untreated reaction vessel.

### 2. Fluorescence Measurement on Untreated Vessel

The two types of reaction vessels prepared in section 1 above were charged with 20 µl of an aqueous solution containing 0.5 µM fluorescent substance (FAM) (fluorescent aqueous solution) and the fluorescence intensity distribution in the circular aperture was measured. Fluorescence detection was carried out with an optical fiber capable of coaxial excitation at 480 nm and detection at 520 nm. This optical fiber scanned along the x-axis across the circular aperture of the reaction vessel through the center of the aperture. Subsequently, 20 µl of mineral oil (Applied Biosystems) was added to the reaction vessel, followed by the same measurement of fluorescence. The results are shown in Fig. 16-1.

When the mineral oil was layered on top of the fluorescent aqueous solution, the fluorescence intensity distribution in the aperture plane of the reaction vessel was altered and the area where maximum fluorescence intensity was obtained (effective area) decreased significantly.

### 3. Fluorescence Measurement on Treated Vessel

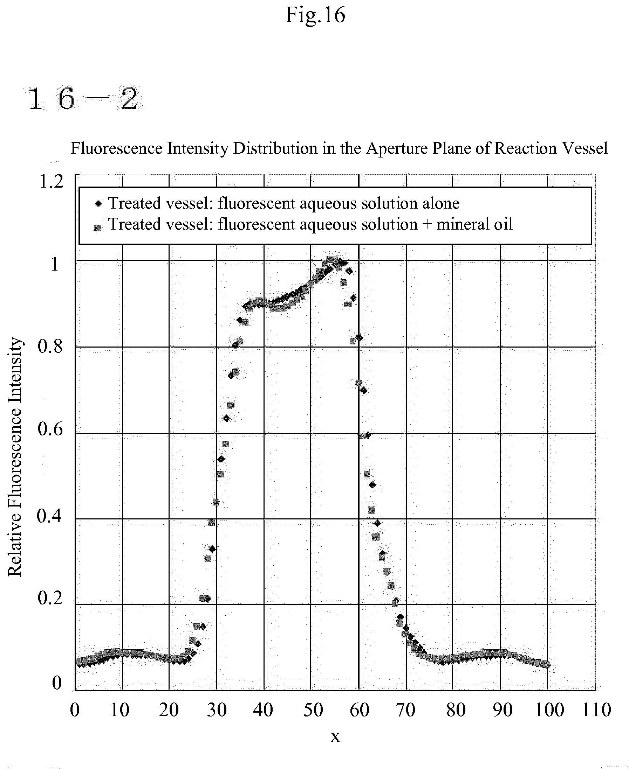

The results of fluorescence measurement performed in the same manner as in section 2 above in the vessels with a low wetting tension that were prepared in section 1 above are shown in Fig. 16-2. The fluorescence intensity distribution in the aperture plane of the reaction vessel was not greatly altered by the addition of mineral oil and the reduction of efficient area observed in the untreated vessel was not recognized.

### INDUSTRIAL APPLICABILITY

The present invention is applicable to such fields as medicine, agriculture, physics, or pharmacology in which genetic analyses are performed using PCR.

### FIGURE LEGENDS

1. Reaction vessel
2. Reaction solution
3. Oily component
4. Coating
10, 70, 80, 180, 280 Specimen testing device
14, 84, 184, 284, 384 Test cartridge container
15, 85, 185, 285 Nozzle head
17, 77, 177, 277 Optical measurement unit
24, 94, 224 Seal
25, 125, 225 Dispenser tip
26, 226 Carrier sealing tip (solid-phase built-in tip)
28, 228 Digital camera
30, 100, 200, 230 Nozzle
92, 192 Cap

## Claims

1. A method of nucleic acid extraction, amplification and detection, comprising the steps of providing a liquid composition which becomes a solid through chemical or thermal changes, overlayering the liquid composition on top of a nucleic acid amplification reaction solution before a nucleic acid amplification reaction, performing the nucleic acid amplification reaction, and solidifying the composition after completion of the nucleic acid amplification reaction,
which uses a nucleic acid amplification reaction vessel where the wetting tension of the inner surface of the reaction vessel is smaller than the surface tension of said composition, wherein the shape of the interface to be formed between the composition and air is level or upwardly convex, and wherein detection of the amplification of the nucleic acid is carried out through the composition by irradiation and/or light reception from above the reaction vessel.

2. The method of claim 1, wherein the wetting tension of the inner surface of the reaction vessel is smaller than 80% of the surface tension of said composition.

3. The method of claim 1, wherein the reaction vessels are made of polytetrafluoroethylene, tetrafluoroethylene/perfluoroalkylvinyl ether copolymer, or tetrafluoroethylene/ethylene copolymer.

4. The method of claim 1, wherein the inner surface of the reaction vessels is coated with a coating agent selected from fluoroacrylic resin, fluorosilane or another fluorine-loaded synthetic resin.

5. An apparatus which performs extraction of nucleic acid from a sample, amplification of the nucleic acid and detection of the nucleic acid in a continuous manner, which apparatus comprises:
(a) means for depositing a liquid composition which becomes solid through chemical or thermal changes;
(b) a nucleic acid reaction vessel where the wetting tension of the inner surface of the reaction vessel is smaller than the surface tension of said composition and where the shape of the interface to be formed between said composition and air is level or upwardly convex; and
(c) an optical measurement unit;
wherein the apparatus is capable of overlayering a liquid composition on top of a nucleic acid amplification reaction solution before a nucleic acid amplification reaction, performing the nucleic acid amplification reaction and solidifying the composition after completion of the nucleic acid amplification reaction, the composition becoming a solid through chemical or thermal changes, and wherein the amplification of the nucleic acid can be optically detected through the composition by irradiation and/or light reception from above the reaction vessel, wherein the reaction vessel contains the nucleic acid amplification reaction solution and the liquid composition overlayered on top of the nucleic acid amplification reaction solution, and wherein the shape of the interface formed between the liquid composition and air is level or upwardly convex.

6. The apparatus according to claim 5, wherein the reaction vessels are made of polytetrafluoroethylene, tetrafluoroethylene/perfluoroalkylvinyl ether copolymer, or tetrafluoroethylene/ethylene copolymer.

7. The apparatus according to claim 5, wherein the inner surface of the reaction vessels is coated with a coating agent selected from fluoroacrylic resin, fluorosilane or another fluorine-loaded synthetic resin.

## Patentansprüche

1. Verfahren zur Extraktion, Amplifikation und Detektion von Nukleinsäure, umfassend die Schritte des Bereitstellens einer flüssigen Zusammensetzung, die durch chemische oder thermische Veränderungen ein Feststoff wird, wobei die flüssige Zusammensetzung die Oberseite einer Nukleinsäure-Amplifikationsreaktionslösung vor einer Nukleinsäure-Amplifikationsreaktion überlagert, zum Ausführen der Nukleinsäure-Amplifikationsreaktion und Verfestigen der Zusammensetzung nach Vervollständigung der Nukleinsäure-Amplifikationsreaktion,
das ein Nukleinsäure-Amplifikationsreaktionsgefäß verwendet, in dem die Benetzungsspannung der Innenfläche des Gefäßes kleiner ist als die Oberflächenspannung der Zusammensetzung, wobei die Form der Schnittstelle, die zwischen der Zusammensetzung und Luft gebildet werden soll, eben oder nach oben konvex ist, und wobei eine Detektion der Amplifikation der Nukleinsäure durch die Zusammensetzung durch Bestrahlung und/oder Lichtempfang von oberhalb des Reaktionsgefäßes durchgeführt wird.

2. Verfahren nach Anspruch 1, wobei die Benetzungsspannung der Innenfläche des Reaktionsgefäßes kleiner als 80 % der Oberflächenspannung der Zusammensetzung ist.

3. Verfahren nach Anspruch 1, wobei die Reaktionsgefäße aus Polytetrafluorethylen, Tetrafluorethylen/Perfluoralkylvinylether-Copolymer oder Tetrafluorethylen/EthylenCopolymer hergestellt sind.

4. Verfahren nach Anspruch 1, wobei die Innenfläche der Reaktionsgefäße mit einem Beschichtungsmittel beschichtet ist, das aus Fluoracrylharz, Fluorsilan oder einem anderen fluorhaltigen synthetischen Harz ausgewählt ist.

5. Vorrichtung, die eine Extraktion von Nukleinsäure aus einer Probe, eine Amplifikation der Nukleinsäure und Detektion der Nukleinsäure auf eine kontinuierliche Weise ausführt, wobei die Vorrichtung folgendes umfasst:
(a) Mittel zum Abscheiden einer flüssigen Zusammensetzung, die durch chemische oder thermische Veränderungen fest wird;
(b) ein Nukleinsäure-Reaktionsgefäß, in dem die Benetzungsspannung der Innenfläche des Reaktionsgefäßes kleiner ist als die Oberflächenspannung der Zusammensetzung und wo die Form der Schnittstelle, die zwischen der Zusammensetzung und Luft gebildet werden soll, eben oder nach oben konvex ist; und
(c) eine optische Messeinheit;
wobei die Vorrichtung in der Lage ist, eine flüssige Zusammensetzung vor einer Nukleinsäure-Amplifikationsreaktion auf die Oberseite einer Nukleinsäure-Amplifikationsreaktionslösung zu schichten, die Nukleinsäure-Amplifikationsreaktion auszuführen und die Zusammensetzung nach Vervollständigung der Nukleinsäure-Amplifikationsreaktion zu verfestigen, wobei die Zusammensetzung durch chemische oder thermische Veränderungen ein Feststoff wird, und wobei die Amplifikation der Nukleinsäure durch die Zusammensetzung durch Bestrahlung und/oder Lichtempfang von oberhalb des Reaktionsgefäßes optisch erfasst werden kann, wobei das Reaktionsgefäß die Nukleinsäure-Amplifikationsreaktionslösung und die flüssige Zusammensetzung, die auf die Oberseite der Nukleinsäure-Amplifikationsreaktionslösung geschichtet ist, enthält, und wobei die Form der Schnittstelle, die zwischen der Zusammensetzung und Luft gebildet werden soll, eben oder nach oben konvex ist.

6. Vorrichtung nach Anspruch 5, wobei die Reaktionsgefäße aus Polytetrafluorethylen, Tetrafluorethylen/Perfluoralkylvinylether-Copolymer oder Tetrafluorethylen/EthylenCopolymer hergestellt sind.

7. Vorrichtung nach Anspruch 5, wobei die Innenfläche der Reaktionsgefäße mit einem Beschichtungsmittel beschichtet ist, das aus Fluoracrylharz, Fluorsilan oder einem anderen fluorhaltigen synthetischen Harz ausgewählt ist.

## Revendications

1. Méthode d'extraction, d'amplification et de détection d'acide nucléique, comprenant les étapes de fourniture d'une composition liquide qui devient un solide par le biais de modifications chimiques ou thermiques, de superposition de la composition liquide au-dessus d'une solution de réaction d'amplification d'acide nucléique avant une réaction d'amplification d'acide nucléique, de réalisation de la réaction d'amplification d'acide nucléique et de solidification de la composition après l'achèvement de la réaction d'amplification d'acide nucléique,
qui utilise un récipient de réaction d'amplification d'acide nucléique où la tension de mouillage de la surface interne du récipient de réaction est inférieure à la tension de surface de ladite composition, la forme de l'interface devant être formée entre la composition et l'air étant plane ou convexe vers le haut, et la détection de l'amplification de l'acide nucléique étant réalisée à travers la composition par rayonnement et/ou réception de lumière depuis le dessus du récipient de réaction.

2. Méthode selon la revendication 1, dans laquelle la tension de mouillage de la surface interne du récipient de réaction est inférieure à 80 % de la tension de surface de ladite composition.

3. Méthode selon la revendication 1, dans laquelle les récipients de réaction sont constitués de polytétrafluoroéthylène, de copolymère tétrafluoroéthylène/perfluoroalkylvinyl-éther ou de copolymère tétrafluoroéthylène/éthylène.

4. Méthode selon la revendication 1, dans laquelle la surface interne des récipients de réaction est recouverte d'un agent de revêtement choisi parmi la résine fluoroacrylique, la résine de fluorosilane ou une autre résine synthétique chargée de fluor.

5. Appareil qui exécute l'extraction d'acide nucléique d'un échantillon, l'amplification de l'acide nucléique et la détection de l'acide nucléique de manière continue, lequel appareil comprend :
(a) un moyen de déposer une composition liquide qui devient solide par le biais de modifications chimiques ou thermiques ;
(b) un récipient de réaction d'acide nucléique où la tension de mouillage de la surface interne du récipient de réaction est inférieure à la tension de surface de ladite composition et où la forme de l'interface devant être formée entre ladite composition et l'air est plane ou convexe vers le haut ; et
(c) une unité de mesure optique ;
l'appareil étant capable de superposer une composition liquide au-dessus d'une solution de réaction d'amplification d'acide nucléique avant une réaction d'amplification de l'acide nucléique, réaliser la réaction d'amplification de l'acide nucléique et solidifier la composition après l'achèvement de la réaction d'amplification de l'acide nucléique, la composition devenant un solide par le biais de modifications chimiques ou thermiques, et l'amplification de l'acide nucléique pouvant être optiquement détectée à travers la composition par rayonnement et/ou réception de lumière depuis le dessus du récipient de réaction, le récipient de réaction contenant la solution de réaction d'amplification de l'acide nucléique et la composition liquide superposée au-dessus de la solution de réaction d'amplification de l'acide nucléique, et la forme de l'interface formée entre la composition liquide et l'air étant plane ou convexe vers le haut.

6. Appareil selon la revendication 5, dans lequel les récipients de réaction sont constitués de polytétrafluoroéthylène, de copolymère tétrafluoroéthylène/perfluoroalkylvinyl-éther ou de copolymère tétrafluoroéthylène/éthylène.

7. Appareil selon la revendication 5, dans lequel la surface interne des récipients de réaction est recouverte d'un agent de revêtement choisi parmi la résine fluoroacrylique, la résine de fluorosilane ou une autre résine synthétique chargée de fluor.
